# EUROPEAN PATENT APPLICATION

(11) **EP 4 793 363 A2**
(43) Date of publication of application: **19.08.2026**
(21) Application number: 26158313.2
(22) Date of filing: 12.02.2026
(51) Int. Cl.: C12Q 1/6848

(54) **AMPLIFICATION METHOD AND REAGENT KIT**

(30) Priority: 13.02.2025 CN 202510164478
(71) Applicant: GeneMind Biosciences Company Limited, Shenzhen City, Guangdong 518023 (CN)
(72) Inventor: XU, Ding, Shenzhen City 518023 (CN); LIU, Lei, Shenzhen City 518023 (CN); WANG, Wen, Shenzhen City 518023 (CN); LIANG, Zehui, Shenzhen City 518023 (CN)
(74) Representative: Schiweck Weinzierl Koch Patentanwälte Partnerschaft mbB

(57) **Abstract**

The present application discloses a variety of amplification methods and reagent kits. In an amplification method provided in the present application, a first cycle of amplification is performed on a target polynucleotide through steps S20-S40, and an extended strand of a first amplification primer is generated on the surface of a solid carrier; then, amplification is performed on the extended strand of the first amplification primer through steps S60-S100, and an extended strand of a second amplification primer is generated on the surface of the solid carrier; subsequently, the extended strand of the first amplification primer and the extended strand of the second amplification primer are used as templates to perform cyclic amplification according to steps S120-S160 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection. Meanwhile, by configuring at least one of betaine having a concentration greater than 0 mol/L and less than or equal to 4 mol/L, Mg²⁺ having a concentration of 0.5-4 mmol/L, NH₄⁺ having a concentration greater than 0 mmol/L and less than or equal to 100 mmol/L, and formamide having a percent concentration by volume (vol/vol% or v/v%) less than or equal to 15% in each of a first solution, a second solution, and a fourth solution, the amplification method provided in the present application can reduce the formation of a secondary structure in the template molecule, improve amplification efficiency, and facilitate the binding of the template molecule to the amplification primer and the extension of the amplification primer, thereby reducing GC bias.

## Description

### TECHNICAL FIELD

The present application relates to the technical field of high-throughput sequencing, and in particular to an amplification method and a reagent kit.

### BACKGROUND

Nucleic acid amplification technology, such as polymerase chain reaction (PCR), has become a mature and standard molecular biology technique and is currently applied across various fields of biomedicine, such as molecular diagnosis, medical testing, and high-throughput sequencing. Through the hybridization and pairing of forward primer and reverse primer with a template DNA molecule, free deoxyribonucleotides (dNTPs) in the system are captured under the action of a polymerase to replicate the template strand, thereby achieving the effect of enriching the template DNA molecules.

Due to the diversity of template DNA molecule sequences, under the same PCR system, the replication efficiency of DNA templates with different sequences often varies. The primary reason is that, according to the principle of complementary base pairing, single-stranded DNA can form an intramolecular secondary structure, particularly DNA molecules with base imbalance (such as extreme GC sequences), which are more prone to forming complex secondary structures. The existence of complex secondary structures affects polymerase extension, and the amplification efficiency of the DNA molecules is significantly reduced, ultimately leading to their relative abundance being much lower than that of other DNA molecules. This is referred to as amplification bias.

In high-throughput sequencing, DNA templates undergo PCR amplification on the surface of a solid carrier. Due to amplification bias, DNA molecules with a high GC content (e.g., a GC content greater than 50%) often have lower amplification efficiency, resulting in smaller and dimmer clusters being generated, with lower detection efficiency; whereas those larger and brighter clusters, such as AT-rich clusters, are more easily detected. This leads to lower abundance, reduced representation, and poorer sequence accuracy for GC-rich DNA templates in the final sequencing results. This effect is referred to as GC bias.

GC bias can lead to a series of problems, for example: (1) when detecting copy number, regions with a high GC content have lower coverage than those with a low GC content, but this does not mean that merely based on sequencing coverage, the copy number of regions with a low GC content is considered higher than that of regions with a high GC content; (2) during RNA sequencing analysis, a lower number of sequencing reads in regions with a high GC content does not necessarily indicate lower expression levels of those genes; (3) during genome assembly, due to the existence of GC bias, regions with a high GC content are sequenced less, making the assembly of these regions more difficult; (4) it is difficult to determine certain sequences within a genome, especially some regions with important functions, such as CpG islands rich in GC in promoter regions; and the like.

Therefore, how to reduce GC bias is a matter worthy of attention.

### SUMMARY

The present application aims to solve at least one of the technical problems existing in the prior art. To this end, the present application provides an amplification method and a reagent kit to reduce GC bias. In one aspect, the present application provides an amplification method, which includes:
S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution or a second solution to obtain an extended strand of the first amplification primer;
S60, replacing the first solution or the second solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution or the second solution to obtain an extended strand of the second amplification primer;
S120, replacing the first solution or the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution or the second solution and taking the extended strand of the first amplification primer and the extended strand of the second amplification primer as templates;
S180, repeating S120-S160 at least once;
where the first solution, the second solution, and the fourth solution all include at least one of betaine, Mg²⁺, NH₄⁺, and formamide, where the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume (vol/vol% or v/v%) less than or equal to 15%.

In the amplification method provided in the present application, a first cycle of amplification is performed on the target polynucleotide through steps S20-S40, and the extended strand of the first amplification primer is generated on the surface of the solid carrier; then, amplification is performed on the extended strand of the first amplification primer through steps S60-S100, and the extended strand of the second amplification primer is generated on the surface of the solid carrier; subsequently, the extended strand of the first amplification primer and the extended strand of the second amplification primer are used as templates to perform cyclic amplification according to steps S120-S160 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection. Meanwhile, by configuring at least one of betaine having a concentration greater than 0 mol/L and less than or equal to 4 mol/L, Mg²⁺ having a concentration of 0.5-4 mmol/L, NH₄⁺ having a concentration greater than 0 mmol/L and less than or equal to 100 mmol/L, and formamide having a percent concentration by volume (vol/vol% or v/v%) less than or equal to 15% in each of the first solution, the second solution, and the fourth solution, the amplification method provided in the present application can reduce the formation of a secondary structure in the template molecule, improve amplification efficiency, and facilitate the binding of the template molecule to the amplification primer and the extension of the amplification primer, thereby reducing GC bias.

In another aspect, the present application provides another amplification method, which includes:
S200, providing a surface of a solid carrier, which includes a first amplification primer, a second amplification primer, and a target polynucleotide that are immobilized thereon, at least a portion of the target polynucleotide complementarily hybridizing with the first amplification primer;
S400, extending the first amplification primer by taking the target polynucleotide as a template and using a first solution or a second solution to obtain an extended strand of the first amplification primer; S600, replacing the first solution or the second solution with a third solution to remove the target polynucleotide, where the third solution contains a denaturing reagent;
S800, replacing the third solution with a fourth solution to allow the extended strand of the first amplification primer to bind to the second amplification primer;
S1000, extending the second amplification primer by taking the extended strand of the first amplification primer as a template and replacing the fourth solution with the first solution or the second solution to obtain an extended strand of the second amplification primer;
S1200, replacing the first solution or the second solution with the third solution;
S1400, replacing the third solution with the fourth solution to allow the extended strand of the first amplification primer and the extended strand of the second amplification primer to bind to the second amplification primer and the first amplification primer, respectively;
S1600, extending the first amplification primer and the second amplification primer by replacing the fourth solution with the first solution or the second solution and taking the extended strand of the first amplification primer and the extended strand of the second amplification primer as templates;
S1800, repeating S1200-S1600 at least once,
where S1600 is performed under a variable-temperature condition, S1200 and S1400 are performed under a constant-temperature condition, and a reaction temperature of S1600 is not lower than reaction temperatures of S1200 and S1400.

In the amplification method provided in the present application, a first cycle of amplification is performed on the target polynucleotide through steps S200-S400, and the extended strand of the first amplification primer is generated on the surface of the solid carrier; then, amplification is performed on the extended strand of the first amplification primer through steps S600-S1000, and the extended strand of the second amplification primer is generated on the surface of the solid carrier; subsequently, the extended strand of the first amplification primer and the extended strand of the second amplification primer are used as templates to perform cyclic amplification according to steps S1200-S1600 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection. Meanwhile, by introducing variable-temperature amplification during the amplification, the amplification method provided in the present application can promote the melting of the secondary structure formed in the template molecule and facilitate the binding of the template molecule to the amplification primer and the extension of the amplification primer, thereby reducing GC bias.

In yet another aspect, the present application provides a yet another amplification method, which includes:
S2000, providing a surface of a solid carrier, which includes a first amplification primer, a second amplification primer, and a target polynucleotide that are immobilized thereon, at least a portion of the target polynucleotide complementarily hybridizing with the first amplification primer;
S4000, extending the first amplification primer by taking the target polynucleotide as a template and using a first solution or a second solution to obtain an extended strand of the first amplification primer;
S6000, replacing the first solution or the second solution with a third solution to remove the target polynucleotide, where the third solution contains a denaturing reagent;
S8000, replacing the third solution with a fourth solution to allow the extended strand of the first amplification primer to bind to the second amplification primer;
S10000, extending the second amplification primer by taking the extended strand of the first amplification primer as a template and replacing the fourth solution with the first solution or the second solution to obtain an extended strand of the second amplification primer;
S12000, replacing the first solution or the second solution with the third solution;
S14000, replacing the third solution with the fourth solution to allow the extended strand of the first amplification primer and the extended strand of the second amplification primer to bind to the second amplification primer and the first amplification primer, respectively;
S16000, extending the first amplification primer and the second amplification primer by replacing the fourth solution with the first solution or the second solution and taking the extended strand of the first amplification primer and the extended strand of the second amplification primer as templates;
S18000, repeating S12000-S16000 at least once,
where in S10000 and S16000, the first solution or the second solution includes dATP, dTTP, dCTP, and dGTP, and a sum of contents of the dATP and the dTTP is less than a sum of contents of the dGTP and the dCTP.

In the amplification method provided in the present application, a first cycle of amplification is performed on the target polynucleotide through steps S2000-S4000, and the extended strand of the first amplification primer is generated on the surface of the solid carrier; then, amplification is performed on the extended strand of the first amplification primer through steps S6000-S 10000, and the extended strand of the second amplification primer is generated on the surface of the solid carrier; subsequently, the extended strand of the first amplification primer and the extended strand of the second amplification primer are used as templates to perform cyclic amplification according to steps S12000-S16000 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection. Meanwhile, by introducing an unbalanced base ratio, i.e., the sum of the contents of dATP and dTTP being less than the sum of the contents of dGTP and dCTP, the amplification method of the present application increases the probability of complementary pairing of dGTP and dCTP with GC bases on the template molecule and preferentially amplifies sequences with a high GC content, thereby enhancing the amplification of GC bases in the template molecule, improving amplification efficiency, and reducing GC bias.

In another aspect, the present application provides a reagent kit, which includes the first solution, the second solution, the third solution, and the fourth solution mentioned in any one of the aspects described above.

The additional aspects and advantages of the present application will be partially set forth in the following description, and will partially become apparent from the following description or be appreciated by practice of the present application.

### BRIEF DESCRIPTION OF THE DRAWINGS

FIG. 1 is a schematic diagram of an amplification method according to an example of the present application;
FIG. 2 is a flowchart of an amplification method according to an example of the present application;
FIG. 3 is a GC bias scatter plot according to an example of the present application;
FIG. 4 is a GC bias scatter plot according to Comparative Example 1-1 of the present application;
FIG. 5 is a GC bias scatter plot according to Comparative Example 1-2 of the present application;
FIG. 6 is a GC bias scatter plot according to Comparative Example 1-3 of the present application;
FIG. 7 is a GC bias scatter plot according to Comparative Example 2-1 of the present application;
FIG. 8 is a GC bias scatter plot according to Comparative Example 3-1 of the present application;
FIG. 9 is a GC bias scatter plot according to Example 4 of the present application;
FIG. 10 is a GC bias scatter plot according to Comparative Example 5-1 of the present application;
FIG. 11 shows comparison curves of constant-temperature amplification and pulsed heating amplification according to Example 6 of the present application;
FIG. 12 is a GC bias scatter plot according to Comparative Example 6-1 of the present application.

### DETAILED DESCRIPTION

In order to make the technical problems, technical solutions, and beneficial effects to be solved by the present application more apparent, the present application will be further described in detail below with reference to the examples. It should be appreciated that the specific examples described herein are intended to illustrate the present application only, rather than limit the present application.

In the present application, the terms used in the examples of the present application are for the purpose of describing particular examples only and are not intended to be limiting to the present application. The term "and/or" describes the associative relationship between associated objects, and refers to three possible relationships. For example, A and/or B may denote that: A is present alone, A and B are present simultaneously, and B is present alone. A and B may be singular or plural. The character "/" generally indicates an "and" relationship between the associated objects.

The term "at least one" means one or more, and "a plurality of" means two or more. "At least one" or similar expressions thereof refer to any combination of these items, including any combination of the singular or plural items. For example, "at least one of a, b, or c" or "at least one of a, b, and c" may each refer to: a, b, c, a-b (i.e., a and b), a-c, b-c, or a-b-c, where a, b, and c may each be a single item or a plurality of items.

As used in the examples and the appended claims of the present application, the singular forms "a", "an", and "the" are intended to include the plural forms as well, unless the context clearly indicates otherwise. The terms "first" and "second" are used for illustrative purposes only, are used for purposes distinguishing features such as substances, orientations, interfaces, messages, requests, and terminals from one another, and should not be construed as indicating or implying relative importance or implicitly indicating the number of the technical features indicated. Therefore, features defined by "first" and "second" may explicitly or implicitly include one or more of the features.

In the description of the present application, the concentration of the related components mentioned herein may not only refer to the specific content of each component, but also refer to the proportional relationship of the contents among components. Therefore, as long as the contents of the related components are scaled up or down according to the examples of the specification in the present application, they shall fall within the scope disclosed in the examples of the specification in the present application.

The abbreviations used herein have their conventional meanings in the chemical and biological fields. The chemical structures and chemical formulas herein are constructed according to standard rules of chemical valence known in the chemical field.

It should be noted that the term "solid carrier" may be any solid support useful for immobilizing nucleic acid sequences, such as nylon membranes, glass slides, plastics, silicon wafers, magnetic beads, and the like.

The term "target polynucleotide" may refer to a single-stranded nucleic acid molecule or nucleic acid analog, e.g., single-stranded DNA, RNA, PNA, LNA, or 2'-O-methRNA. The source of the target polynucleotide may be, for example, an animal, a plant, a microorganism, etc.

The term "amplification primer" may be an oligonucleotide or a nucleic acid molecule that hybridizes with a target polynucleotide of interest. In the embodiments, the amplification primer serves as a substrate onto which nucleotides or nucleotide analogs may be polymerized by a polymerase. For example, the amplification primer may be used as a starting point for DNA or RNA synthesis. For example, the amplification primer may hybridize with a target polynucleotide to form a hybrid complex so as to trigger the synthesis of a new strand complementary to the target polynucleotide. The amplification primer may include any combination of nucleotides or analogs thereof. Illustratively, the amplification primer may be a single-stranded oligonucleotide or polynucleotide.

The term "denaturing reagent" may refer to chemical substances capable of disrupting the double-stranded structure in a nucleic acid molecule to change it from double-stranded to single-stranded. These chemical substances can achieve the denaturation of double-stranded nucleic acid molecules by disrupting the hydrogen bonds between the strands of the nucleic acid molecules. Illustratively, the denaturing reagent may be, for example, a strong acid (e.g., HCl), a strong base (e.g., NaOH), urea, methanol, ethanol, an amide compound, guanidine hydrochloride, guanidine isothiocyanate, or the like. The term "dNTPs" refers to deoxyribonucleoside triphosphates, which include a phosphate group, deoxyribose, and a nitrogenous base (one of A, T, C, and G), and generally include four types: dATP, dGTP, dTTP, and dCTP, which represent deoxyadenosine triphosphate, deoxyguanosine triphosphate, deoxythymidine triphosphate, and deoxycytidine triphosphate, respectively.

Generally, a clone of a template molecule can be generated by amplifying the template molecule. The template molecule amplified multiple times can generate multiple clones and form a cluster, i.e., an amplification cluster. The amplification cluster can amplify the signals generated when nucleotides or nucleotide analogs are incorporated into the template molecule during sequencing, thereby making it easier for the detection system to detect the signals and improving the efficiency and accuracy of signal detection. However, during amplification, a template molecule may form an intramolecular secondary structure due to self-folding, especially a template molecule with a high GC content, which is more prone to forming a complex secondary structure during amplification. Additionally, since paired GC contains three hydrogen bonds therebetween while paired AT contains two hydrogen bonds therebetween, the secondary structure formed in the template molecule with a high GC content is more difficult to melt relative to the secondary structure formed in the template molecule with a low GC content. The secondary structure in the template molecule negatively affects the binding between the template molecule and the amplification primer as well as the extension of the primer. This reduces amplification efficiency, resulting in fewer generated clones and a smaller amplification cluster. Due to the smaller amplification cluster, the signals generated when nucleotides or nucleotide analogs are incorporated into the template molecule during sequencing are weaker. Consequently, the detection system struggles to detect the signals, leading to lower efficiency and accuracy in signal detection. As a result, in the final obtained sequencing results, the abundance of the template molecule with a high GC content is low relative to the abundance of the template molecule with a low GC content. This effect is commonly referred to as GC bias.

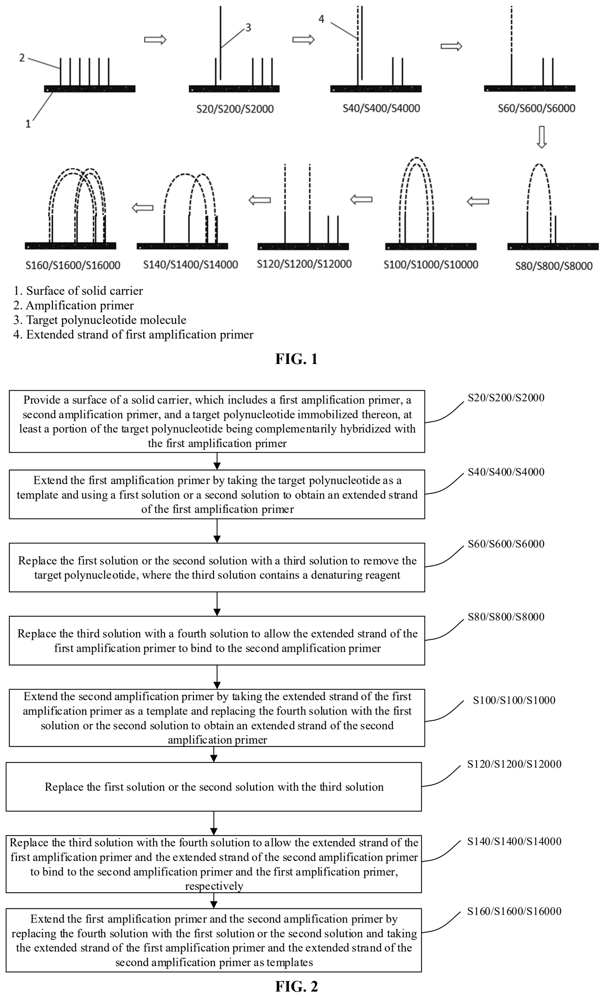

To improve amplification efficiency and reduce GC bias, the inventors of the present application have conducted long-term exploration and research, leading to the development of a variety of amplification methods. As shown in FIG. 1 and FIG. 2, an amplification method provided in the embodiments of the present application includes:
S20, providing a surface of a solid carrier, which includes a first amplification primer, a second amplification primer, and a target polynucleotide that are immobilized thereon, at least a portion of the target polynucleotide complementarily hybridizing with the first amplification primer.

The surface of the solid carrier may be the surface of a sequencing chip. Illustratively, the surface of the solid carrier includes a plurality of nanopores arranged in an array. The size of the nanopores and the pore spacing distance may be determined according to actual conditions. The first amplification primer and the second amplification primer are immobilized on the inner surface of the nanopore by surface chemical modification. The first amplification primer and the second amplification primer are, for example, single-stranded oligonucleotides. The first amplification primer and the second amplification primer can be immobilized on the surface of the solid carrier by means known in the art, such as covalent bonding or physical adsorption. The target polynucleotide may be, for example, a single-stranded nucleic acid molecule or a nucleic acid analogue, and at least a portion of one terminus of the target polynucleotide complementarily hybridizes with the first amplification primer in the manner of base complementary pairing to form a hybrid complex, thereby immobilizing the target polynucleotide on the surface of the solid carrier. The first amplification primer that complementarily hybridizes with the target polynucleotide can serve as a starting point for synthesizing the complementary strand of the target polynucleotide, and under the action of a DNA polymerase and conditions suitable for polymerase chain reaction, it initiates the synthesis of the complementary strand of the target polynucleotide.

As shown in FIG. 1 and FIG. 2, the amplification method provided in the embodiments of the present application further includes:
S40, extending the first amplification primer by taking the target polynucleotide as a template and using a first solution or a second solution to obtain an extended strand of the first amplification primer.

The first solution or the second solution contains components required for extension, such as DNA polymerases, nucleotides (e.g., A, T, C, G), or nucleotide analogs. Under the action of a DNA polymerase and conditions suitable for polymerase chain reaction, nucleotides or nucleotide analogs are bound to the 3' terminus of the first amplification primer by taking the target polynucleotide as a template, thereby extending the first amplification primer. The obtained extended strand of the first amplification primer is the complementary strand of the target polynucleotide. Illustratively, the DNA polymerase is selected from one or more of rTaq, Canace, Pfu, KOD, Phusion, Primerstar, Tth, BST, and BSU. Preferably, the DNA polymerase has a concentration of 4 U/mL.

As shown in FIG. 1, the amplification method provided in the embodiments of the present application further includes:
S60, replacing the first solution or the second solution with a third solution to remove the target polynucleotide, where the third solution contains a denaturing reagent.

By replacing the reacted first solution or second solution with the third solution, the denaturing reagent in the third solution can be used to weaken or disrupt the molecular force between the target polynucleotide and the complementary strand thereof, for example, to break the hydrogen bond between the target polynucleotide and the complementary strand thereof, so that the double-stranded molecules are melted to form single-stranded molecules, thereby removing the target polynucleotide. Illustratively, the denaturing reagent may be, for example, a strong acid (e.g., HCl), a strong base (e.g., NaOH), urea, methanol, ethanol, an amide compound, guanidine hydrochloride, guanidine isothiocyanate, or the like. Preferably, the denaturing reagent is an amide compound, such as formamide.

As shown in FIG. 1, the amplification method provided in the embodiments of the present application further includes:
S80, replacing the third solution with a fourth solution to allow the extended strand of the first amplification primer to bind to the second amplification primer.

The free terminus of the newly synthesized extended strand of the first amplification primer includes a portion capable of complementarily hybridizing with the second amplification primer. The fourth solution contains components required for hybridization. Illustratively, the fourth solution may be the first solution or the second solution without the DNA polymerase and the nucleotides or nucleotide analogs. By replacing the third solution with the fourth solution, the free terminus of the extended strand of the first amplification primer can hybridize with the second amplification primer on the surface of the solid carrier, so as to extend the second amplification primer by taking the extended strand of the first amplification primer as a template to obtain the extended strand of the second amplification primer, i.e., the complementary strand of the extended strand of the first amplification primer.

As shown in FIG. 1, the amplification method provided in the embodiments of the present application further includes:
S100, extending the second amplification primer by taking the extended strand of the first amplification primer as a template and replacing the fourth solution with the first solution or the second solution to obtain an extended strand of the second amplification primer.

The first solution or the second solution contains components required for extension, such as DNA polymerases, nucleotides, or nucleotide analogs. Under the action of a DNA polymerase and conditions suitable for polymerase chain reaction, nucleotides or nucleotide analogs are bound to the 3' terminus of the second amplification primer by taking the extended strand of the first amplification primer as a template, thereby extending the second amplification primer. The obtained extended strand of the second amplification primer is the complementary strand of the extended strand of the first amplification primer. As shown in FIG. 1, the amplification method provided in the embodiments of the present application further includes:
S120, replacing the first solution or the second solution with the third solution.

The third solution contains a denaturing reagent. By replacing the reacted first solution or second solution with the third solution, the denaturing reagent in the third solution can be used to weaken or disrupt the molecular force between the extended strand of the first amplification primer and the extended strand of the second amplification primer, for example, to break the hydrogen bond between the extended strand of the first amplification primer and the extended strand of the second amplification primer, so that the double-stranded molecules are melted to form single-stranded molecules.

The amplification method provided in the embodiments of the present application further includes:
S140, replacing the third solution with the fourth solution to allow the extended strand of the first amplification primer and the extended strand of the second amplification primer to bind to the second amplification primer and the first amplification primer, respectively.

The free terminus of the extended strand of the first amplification primer includes a portion capable of complementarily hybridizing with the second amplification primer, and the free terminus of the extended strand of the second amplification primer includes a portion capable of complementarily hybridizing with the first amplification primer. The fourth solution contains components required for hybridization. Illustratively, the fourth solution may be the first solution or the second solution without the DNA polymerase and the nucleotides or nucleotide analogs. By replacing the third solution with the fourth solution, the free terminus of the extended strand of the first amplification primer can hybridize with the second amplification primer on the surface of the solid carrier, and the free terminus of the extended strand of the second amplification primer can hybridize with the first amplification primer on the surface of the solid carrier.

The amplification method provided in the embodiments of the present application further includes:
S160, extending the first amplification primer and the second amplification primer by replacing the fourth solution with the first solution or the second solution and taking the extended strand of the first amplification primer and the extended strand of the second amplification primer as templates.

The first solution or the second solution contains components required for extension, such as DNA polymerases, nucleotides, or nucleotide analogs. Under the action of a DNA polymerase and conditions suitable for polymerase chain reaction:
nucleotides or nucleotide analogs are bound to the 3' terminus of the second amplification primer by taking the extended strand of the first amplification primer as a template to extend the second amplification primer, thereby obtaining an extended strand of the second amplification primer, which is the complementary strand of the extended strand of the first amplification primer, i.e., the target polynucleotide; and
nucleotides or nucleotide analogs are bound to the 3' terminus of the first amplification primer by taking the extended strand of the second amplification primer as a template to extend the first amplification primer, thereby obtaining an extended strand of the first amplification primer, which is the complementary strand of the extended strand of the second amplification primer, i.e., the complementary strand of the target polynucleotide.

The amplification method provided in the embodiments of the present application further includes:
S180, repeating S120-S160 at least once.

As such, multiple target polynucleotides can be obtained, i.e., forming a molecular cluster of target polynucleotides.

In the amplification method provided in the present application, a first cycle of amplification is performed on the target polynucleotide through steps S20-S40, and the extended strand of the first amplification primer is generated on the surface of the solid carrier; then, amplification is performed on the extended strand of the first amplification primer through steps S60-S100, and the extended strand of the second amplification primer is generated on the surface of the solid carrier; subsequently, the extended strand of the first amplification primer and the extended strand of the second amplification primer are used as templates to perform cyclic amplification according to steps S120-S160 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection.

In the embodiments of the present application, the first solution, the second solution, and the fourth solution all further include at least one of betaine, Mg²⁺, NH₄⁺, and formamide, where the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume (vol/vol% or v/v%) less than or equal to 15%. By configuring at least one of betaine having a concentration greater than 0 mol/L and less than or equal to 4 mol/L, Mg²⁺ having a concentration of 0.5-4 mmol/L, NH₄⁺ having a concentration greater than 0 mmol/L and less than or equal to 100 mmol/L, and formamide having a percent concentration by volume (vol/vol% or v/v%) less than or equal to 15% in each of the first solution, the second solution, and the fourth solution, the amplification method provided in the present application can reduce the formation of a secondary structure in the template molecule, improve amplification efficiency, and facilitate the binding of the template molecule to the amplification primer and the extension of the amplification primer, thereby reducing GC bias.

Specifically, the inventors of the present application have found through research that betaine can reduce the formation of secondary structures in the template molecule due to GC enrichment, improve the amplification efficiency of GC-rich sequences, and thus reduce GC bias. The inventors believe this may be attributed to betaine's ability to increase hydration in guanine- and cytosine-rich regions of the template molecule, influencing the structure of the template molecule and changing its flexibility. This facilitates DNA polymerase extension along the template molecule, thereby reducing the formation of secondary structures in the template molecule, improving amplification efficiency, and reducing GC bias. Furthermore, betaine can lower the melting temperature of sequences rich in GC content, facilitating more uniform denaturation of double-stranded molecules and reducing melting stability variations caused by differences in GC content, thereby reducing GC bias. Additionally, betaine can stabilize template molecule-protein complexes, helping to maintain the stability of template molecules during amplification and reducing polymerase dissociation induced by GC-rich regions. The inventors of the present application have also found through research that betaine exerts the effects described above or produces the outcomes described above within a certain concentration range. If the concentration of betaine exceeds this range, the amplification is inhibited.

In some embodiments, the betaine has a concentration greater than 0 mol/L and less than or equal to 3 mol/L in the first solution. Illustratively, the betaine may have a concentration of 0.5 mol/L, 1 mol/L, 1.5 mol/L, 2 mol/L, 2.5 mol/L, 3 mol/L, or a concentration between any two of these values in the first solution.

In some embodiments, the betaine has a concentration of 1.5-2.5 mol/L in the second solution. Illustratively, the betaine may have a concentration of 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or a concentration between any two of these values in the second solution.

In some embodiments, the betaine has a concentration of 1.5-2.5 mol/L in the fourth solution. Illustratively, the betaine may have a concentration of 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or a concentration between any two of these values in the fourth solution.

The inventors of the present application have also found through research that Mg²⁺ can be used as an activity additive of the polymerase and is conducive to activating the activity of the polymerase, so that the polymerase catalyzes the formation of a phosphodiester bond between the 3'-OH of the amplification primer bound to the template molecule and the phosphate group of the nucleotide or nucleotide analog, thereby binding the nucleotide or nucleotide analog to the amplification primer and binding the nucleotide or nucleotide analog to the template molecule in the manner of base complementary pairing, that is, the nucleotide or nucleotide analog is incorporated into the template molecule. This improves amplification efficiency and reduces GC bias, thereby helping to minimize the intensity differences in signals generated during sequencing between molecular clusters of target polynucleotides with high AT and high GC and improving the sequencing accuracy. In addition, the concentration of Mg²⁺ is also critical to the amplification. An excessively low concentration of Mg²⁺ can lead to failure of amplification, and an excessively high concentration can lead to increased base mismatches during amplification. Therefore, a suitable Mg²⁺ concentration should be selected.

In some embodiments, the Mg²⁺ may have a concentration of 0.5 mmol/L, 1 mmol/L, 1.5 mmol/L, 2 mmol/L, 2.5 mmol/L, 3 mmol/L, 3.5 mmol/L, 4 mmol/L, or a concentration between two of the values described above.

In some embodiments, in S40, the first amplification primer is extended by using the first solution, the Mg²⁺ has a concentration of 2-4 mmol/L in the first solution, and the betaine has a concentration of 0.1-2 mol/L in the first solution. In the first cycle of amplification, the synergistic use of a higher concentration of Mg²⁺ and a lower concentration of betaine can improve amplification efficiency, reduce GC bias, and facilitate the extension of the first amplification primer to generate the extended strand of the first amplification primer.

In S100 or S160, the fourth solution is replaced with the second solution, the Mg²⁺ has a concentration of 2-4 mmol/L in the second solution, and the betaine has a concentration of 0.1-2 mol/L in the second solution; or in S100 or S160, the fourth solution is replaced with the second solution, the Mg²⁺ has a concentration of 1-1.5 mmol/L in the second solution, and the betaine has a concentration of 2-3 mol/L in the second solution.

The inventors of the present application have found that during the extension of the first amplification primer and/or the second amplification primer of S100 or S160, replacing the fourth solution with the second solution containing 2-4 mmol/L Mg²⁺ and 0.1-2 mol/L betaine, or replacing the fourth solution with the second solution containing 1-1.5 mmol/L Mg²⁺ and 2-3 mol/L betaine can effectively extend the first amplification primer and/or the second amplification primer, thereby generating the extended strand of the first amplification primer and/or the extended strand of the second amplification primer. The inventors speculate that this may be related to the improved amplification efficiency and reduced GC bias achieved in S40 through the synergistic use of a higher concentration of Mg²⁺ and a lower concentration of betaine, thereby providing more feasible schemes for subsequent amplification steps. In some embodiments, in S40, the first amplification primer is extended by using the second solution, the Mg²⁺ has a concentration of 2-4 mmol/L in the second solution, and the betaine has a concentration of 0.1-2 mol/L in the second solution. In the first cycle of amplification, the synergistic use of a higher concentration of Mg²⁺ and a lower concentration of betaine can improve amplification efficiency, reduce GC bias, and facilitate the extension of the first amplification primer to generate the extended strand of the first amplification primer.

In S100 or S160, the fourth solution is replaced with the first solution, the Mg²⁺ has a concentration of 2-4 mmol/L in the first solution, and the betaine has a concentration of 0.1-2 mol/L in the first solution; or in S100 or S160, the fourth solution is replaced with the first solution, the Mg²⁺ has a concentration of 1-1.5 mmol/L in the first solution, and the betaine has a concentration of 2-3 mol/L in the first solution. The inventors of the present application have found that during the extension of the first amplification primer and/or the second amplification primer of S100 or S160, replacing the fourth solution with the first solution containing 2-4 mmol/L Mg²⁺ and 0.1-2 mol/L betaine, or replacing the fourth solution with the first solution containing 1-1.5 mmol/L Mg²⁺ and 2-3 mol/L betaine can effectively extend the first amplification primer and/or the second amplification primer, thereby generating the extended strand of the first amplification primer and/or the extended strand of the second amplification primer. The inventors speculate that this may be related to the improved amplification efficiency and reduced GC bias achieved in S40 through the synergistic use of a higher concentration of Mg²⁺ and a lower concentration of betaine, thereby providing more feasible schemes for subsequent amplification steps. In some embodiments, in S40, the first amplification primer is extended by using the first solution, the Mg²⁺ has a concentration of 2-4 mmol/L in the first solution, and the betaine has a concentration of 0.1-2 mol/L in the first solution; in S100 or S160, the fourth solution is replaced with the second solution, the Mg²⁺ has a concentration of 2-4 mmol/L in the second solution, and the betaine has a concentration of 0.1-2 mol/L in the second solution; or
in S40, the first amplification primer is extended by using the second solution, the Mg²⁺ has a concentration of 2-4 mmol/L in the second solution, and the betaine has a concentration of 0.1-2 mol/L in the second solution; in S100 or S160, the fourth solution is replaced with the first solution, the Mg²⁺ has a concentration of 2-4 mmol/L in the first solution, and the betaine has a concentration of 0.1-2 mol/L in the first solution;
the first solution or the second solution further includes dNTPs, the dNTPs including dATP, dTTP, dCTP, and dGTP, and a sum of contents of dATP and dTTP being equal to a sum of contents of dGTP and dCTP. The amplification primer is extended by using the first solution or the second solution. Particularly, in S40, a base solution with a balanced ratio, i.e., the sum of the contents of dATP and dTTP being equal to the sum of the contents of dGTP and dCTP, is configured in the first solution or the second solution, and this facilitates the amplification of all target polynucleotide molecules as much as possible. Illustratively, dATP, dTTP, dCTP, and dGTP may be present at equal and balanced concentrations; for example, each of dATP, dTTP, dCTP, and dGTP may have a concentration of 200 µmol/L, 100 µmol/L, 50 µmol/L, 10 µmol/L, 5 µmol/L, 1 µmol/L, or the like.

In some embodiments, in S40, the first amplification primer is extended by using the first solution, the Mg²⁺ has a concentration of 2-4 mmol/L in the first solution, and the betaine has a concentration of 0.1-2 mol/L in the first solution; in S100 or S160, the fourth solution is replaced with the second solution, the Mg²⁺ has a concentration of 1-1.5 mmol/L in the second solution, and the betaine has a concentration of 2-3 mol/L in the second solution; or
in S40, the first amplification primer is extended by using the second solution, the Mg²⁺ has a concentration of 2-4 mmol/L in the second solution, and the betaine has a concentration of 0.1-2 mol/L in the second solution; in S100 or S160, the fourth solution is replaced with the first solution, the Mg²⁺ has a concentration of 1-1.5 mmol/L in the first solution, and the betaine has a concentration of 2-3 mol/L in the first solution;
the second solution or the first solution further includes dNTPs, the dNTPs including dATP, dTTP, dCTP, and dGTP, and a sum of contents of the dATP and the dTTP being less than a sum of contents of the dGTP and the dCTP. The amplification primer is extended by using the first solution or the second solution, and particularly, in S100 or S160, preparing a base solution with an imbalanced ratio, i.e., a sum of the contents of dATP and dTTP being less than a sum of the contents of dGTP and dCTP, in the first solution or the second solution is beneficial to increasing the probability of complementary pairing of dGTP and dCTP with GC bases on the template molecule and preferentially amplifying sequences with a high GC content, thereby enhancing the amplification of GC bases in the template molecule, improving amplification efficiency, and reducing GC bias. Illustratively, the sum of the contents of dATP and dTTP is 90%, 80%, 70%, 60%, 50%, 40%, 30%, 20%, 10%, 5%, or the like of the sum of the contents of dGTP and dCTP.

The inventors of the present application have also found through research that NH₄⁺ can improve the reactivity of the polymerase, thereby improving amplification efficiency and reducing GC bias. The inventors speculate that the increase in the reactivity of the polymerase by NH₄⁺ may be associated with the ability of NH₄⁺ to change the charge distribution on the surface of the polymerase and/or to change the spatial configuration of the polymerase, such that the polymerase can be more easily bound to the reaction sites of the template molecule and/or the amplification primer and catalyzes the formation of a phosphodiester bond between the 3'-OH of the amplification primer and the phosphate group of the nucleotide or nucleotide analog, thereby binding the nucleotide or nucleotide analog to the amplification primer and binding the nucleotide or nucleotide analog to the nucleic acid template via complementary base pairing, i.e., incorporating the nucleotide or nucleotide analog into the template molecule.

In some embodiments, the NH₄⁺ may have a concentration of 0.1 mmol/L, 1 mmol/L, 5 mmol/L, 10 mmol/L, 20 mmol/L, 30 mmol/L, 40 mmol/L, 50 mmol/L, 60 mmol/L, 70 mmol/L, 80 mmol/L, 90 mmol/L, 100 mmol/L, or a concentration between any two of these values.

In some embodiments, the NH₄⁺ is NH₄⁺ provided by at least one of ammonium sulfate, ammonium chloride, and ammonium acetate. That is, the first solution, the second solution, and the fourth solution of the present application all include at least one of ammonium sulfate, ammonium chloride, and ammonium acetate, or at least one of ammonium sulfate, ammonium chloride, and ammonium acetate is added when the first solution, the second solution, and the fourth solution are prepared.

In addition, the inventors of the present application have also found that formamide can be used as a denaturing reagent, and can reduce the stability of double-stranded molecules in the secondary structure in the template molecule, disrupt the hydrogen bonds between the double-stranded molecules, cause melting of the double-stranded molecules at a relatively low temperature, and facilitate the binding of the template molecule to the amplification primer, which facilitates the amplification, thereby improving amplification efficiency and reducing GC bias. This difference in effect is mainly related to the concentration of formamide in the solution. For example, when the percent concentration by volume (vol/vol% or v/v%) of formamide reaches or exceeds 60%, e.g., 60-100%, formamide is used as a denaturing reagent, and when the percent concentration by volume (vol/vol% or v/v%) of formamide is greater than or equal to 3% and less than or equal to 15%, formamide can reduce the stability of double-stranded molecules in the secondary structure in the template molecule, disrupt the hydrogen bonds between the double-stranded molecules, cause melting of the double-stranded molecules at a relatively low temperature, and facilitate the binding of the template molecule to the amplification primer, which facilitates the amplification, thereby improving amplification efficiency and reducing GC bias. Therefore, by controlling the concentration of formamide, the formamide can be controlled to exert different effects and achieve different purposes.

In some embodiments, the formamide may have a percent concentration by volume (vol/vol% or v/v%) of 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, or a percent concentration by volume (vol/vol% or v/v%) between any two of these values in the first solution, the second solution, and the fourth solution.

In the embodiments of the present application, the first solution, the second solution, and the fourth solution all include DMSO (dimethyl sulfoxide), and the DMSO has a percent concentration by volume (vol/vol% or v/v%) of 1-5% in each of the first solution, the second solution, and the fourth solution.

Since sequences with a high GC content in the template molecule are often difficult to amplify due to the easy formation of secondary structures, DMSO can improve the spatial configuration of the template molecule with a high GC content, reduce the formation of secondary structures, and enable the polymerase to extend along the template molecule, thereby improving amplification efficiency and reducing GC bias.

In some embodiments, the DMSO may have a percent concentration by volume (vol/vol% or v/v%) of 1%, 1.5%, 2%, 2.5%, 3%, 3.5%, 4%, 4.5%, 5%, or a concentration between any two of these values in each of the first solution, the second solution, and the fourth solution.

In the embodiments of the present application, the first solution, the second solution, and the fourth solution further include Trisbase (tris(hydroxymethyl)aminomethane), and the Trisbase has a concentration of 10-500 mmol/L in each of the first solution, the second solution, and the fourth solution. The Trisbase can provide an appropriate buffer environment and ionic strength for amplification.

In some embodiments, the Trisbase may have a concentration of 10 mmol/L, 15 mmol/L, 20 mmol/L, 25 mmol/L, 30 mmol/L, 35 mmol/L, 40 mmol/L, 45 mmol/L, 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, 200 mmol/L, 210 mmol/L, 220 mmol/L, 230 mmol/L, 240 mmol/L, 250 mmol/L, 260 mmol/L, 270 mmol/L, 280 mmol/L, 290 mmol/L, 300 mmol/L, 310 mmol/L, 320 mmol/L, 330 mmol/L, 340 mmol/L, 350 mmol/L, 360 mmol/L, 370 mmol/L, 380 mmol/L, 390 mmol/L, 400 mmol/L, 410 mmol/L, 420 mmol/L, 430 mmol/L, 440 mmol/L, 450 mmol/L, 460 mmol/L, 470 mmol/L, 480 mmol/L, 490 mmol/L, 500 mmol/L, or a concentration between any two of these values in the first solution, the second solution, and the fourth solution.

In the embodiments of the present application, the first solution, the second solution, and the fourth solution further include a single-stranded binding protein and/or bovine serum albumin. The single-stranded binding protein and/or bovine serum albumin can protect the DNA polymerase and effectively promote the amplification of the template molecule and reduce GC bias. The single-stranded binding protein has a concentration of 0.01-1 mg/mL in the first solution, the second solution, and the fourth solution; the bovine serum albumin has a percent concentration by volume (vol/vol% or v/v%) of 0.05-2% in the first solution, the second solution, and the fourth solution. Illustratively, the single-stranded binding protein may have a concentration of 0.01 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.7 mg/mL, 0.75 mg/mL, 0.8 mg/mL, 0.85 mg/mL, 0.9 mg/mL, 0.95 mg/mL, 1 mg/mL, or a concentration between any two of these values in the first solution, the second solution, and the fourth solution. The bovine serum albumin may have a percent concentration by volume (vol/vol% or v/v%) of 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.05%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, or a concentration between any two of these values in the first solution, the second solution, and the fourth solution.

In the embodiments of the present application, the first solution, the second solution, and the fourth solution further include 10-50 mmol/L tetramethylammonium chloride or tetraethylammonium chloride. Although the inventors are not aware of the reason, through research, tetramethylammonium chloride or tetraethylammonium chloride is found to be able to facilitate the amplification of the template molecule, thereby reducing GC bias. Illustratively, the tetramethylammonium chloride or tetraethylammonium chloride may have a concentration of 10 mmol/L, 20 mmol/L, 30 mmol/L, 40 mmol/L, 50 mmol/L, 60 mmol/L, or a concentration between any two of these values.

In the embodiments of the present application, the first solution, the second solution, and the fourth solution further include a surfactant having a percent concentration by volume (vol/vol% or v/v%) of 0.01-2%. Illustratively, the surfactant is a nonionic surfactant, for example, selected from one or more of Tween 20, DD (N,N-dimethyldodecylamine N-oxide), DDM (n-dodecyl-β-D-maltoside), and Triton X-100 (polyethylene glycol octylphenyl ether).

In some embodiments, the surfactant may have a concentration of 0.01%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.05%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, or a concentration between any two of these values.

In the embodiments of the present application, S160 is performed under a variable-temperature condition, S120 and S140 are performed under a constant-temperature condition, and a reaction temperature of S160 is not lower than reaction temperatures of S120 and S140.

The inventors of the present application have found through research that the temperature can affect the stability of the secondary structure formed due to high GC content in the template molecule, and a higher temperature is more likely to promote melting of the secondary structure in the template molecule. Based on this finding, the present application promotes the melting of the secondary structure formed due to high GC content in the template molecule by introducing variable-temperature amplification during the constant-temperature amplification, thereby facilitating the binding of the template molecule to the amplification primer and the extension of the amplification primer, improving amplification efficiency, and reducing GC bias.

Specifically, S160 is performed under a variable-temperature condition, including:
in S160, performing a first heating on a reaction system of S160 to raise the temperature of the reaction system of S160 to a first temperature, and maintaining the first temperature for a first preset duration;
after the first preset duration, performing a second heating on the reaction system of S160 to raise the temperature of the reaction system of S160 from the first temperature to a second temperature, and
maintaining the second temperature for a second preset duration.

Since the temperature at which the polymerase exhibits optimal activity may be different from the temperature at which the secondary structure melts, the use of a single temperature to complete amplification may not give consideration to both the activity of the polymerase and the melting effect of the secondary structure. Therefore, the present application creatively proposes adopting the above gradient heating for the reaction system of S160, which can balance the activity of the polymerase in the reaction system and the promoting effect on the melting of the secondary structure formed in the template molecule. For example, supposing that the polymerase exhibits the optimal activity at the first temperature, the temperature, however, does not promote the melting of secondary structures. Therefore, it is conceivable to raise the temperature of the reaction system to a second temperature on the basis of the first temperature and maintain the second temperature for a second preset duration after the first preset duration. As such, the polymerase can maintain good activity to continue to exert its functions, while in terms of the secondary structure in the template molecule, the separation of the two strands is promoted, which facilitates the binding of the template molecule to the amplification primer and the extension of the amplification primer, thereby improving amplification efficiency and reducing GC bias. In some embodiments, the first temperature is 60 °C-65 °C, and the first preset duration is 1-10 s; and/or the second temperature is 80 °C-85 °C, and the second preset duration is 1-10 s. Illustratively, the first temperature may be 60 °C, 61 °C, 62 °C, 63 °C, 64 °C, 65 °C, or a temperature between any two of these values. The first preset duration may be 1 s, 2 s, 3 s, 4 s, 5 s, 6 s, 7 s, 8 s, 9 s, 10 s, or a duration between any two of these values. The second temperature may be 80 °C, 81 °C, 82 °C, 83 °C, 84 °C, 85 °C, or a temperature between any two of these values. The second preset duration may be 1 s, 2 s, 3 s, 4 s, 5 s, 6 s, 7 s, 8 s, 9 s, 10 s, or a duration between any two of these values.

In some embodiments, the first heating rate and the second heating rate are 0.1-5.0 °C/s. Illustratively, the first heating rate and the second heating rate may be 0.1 °C/s, 0.2 °C/s, 0.3 °C/s, 0.4 °C/s, 0.5 °C/s, 0.6 °C/s, 0.7 °C/s, 0.8 °C/s, 0.9 °C/s, 1 °C/s, 1.1 °C/s, 1.2 °C/s, 1.3 °C/s, 1.4 °C/s, 1.5 °C/s, 1.6 °C/s, 1.7 °C/s, 1.8 °C/s, 1.9 °C/s, 2 °C/s, 2.1 °C/s, 2.2 °C/s, 2.3 °C/s, 2.4 °C/s, 2.5 °C/s, 2.6 °C/s, 2.7 °C/s, 2.8 °C/s, 2.9 °C/s, 3 °C/s, 3.1 °C/s, 3.2 °C/s, 3.3 °C/s, 3.4 °C/s, 3.5 °C/s, 3.6 °C/s, 3.7 °C/s, 3.8 °C/s, 3.9 °C/s, 4 °C/s, 4.1 °C/s, 4.2 °C/s, 4.3 °C/s, 4.4 °C/s, 4.5 °C/s, 4.6 °C/s, 4.7 °C/s, 4.8 °C/s, 4.9 °C/s, 5 °C/s, or a rate between any two of these values.

In some embodiments, S160 is performed under a variable-temperature condition, including:
in S160, performing at least one pulsed heating on the reaction system of S160 to raise the temperature of the reaction system of S160 to a third temperature, where the duration of one pulse is a third preset duration.

By adopting pulsed heating, hydrogen bonds between GCs in the secondary structure formed in the template molecule can be more effectively disrupted, so that the secondary structure is more susceptible to melting, which facilitates the binding of the template molecule to the amplification primer and the extension of the amplification primer, thereby improving amplification efficiency and reducing GC bias.

In some embodiments, the third temperature is 60 °C-85 °C, and the third preset duration is 1-10 s. The third temperature may be 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, 75 °C, 76 °C, 77 °C, 78 °C, 79 °C, 80 °C, 81 °C, 82 °C, 83 °C, 84 °C, 85 °C, or a temperature between any two of these values. The third preset duration may be 1 s, 2 s, 3 s, 4 s, 5 s, 6 s, 7 s, 8 s, 9 s, 10 s, or a duration between any two of these values.

In some embodiments, the pulsed heating has a heating rate of 0.1-5.0 °C/s. The pulsed heating may have a heating rate of 0.1 °C/s, 0.2 °C/s, 0.3 °C/s, 0.4 °C/s, 0.5 °C/s, 0.6 °C/s, 0.7 °C/s, 0.8 °C/s, 0.9 °C/s, 1 °C/s, 1.1 °C/s, 1.2 °C/s, 1.3 °C/s, 1.4 °C/s, 1.5 °C/s, 1.6 °C/s, 1.7 °C/s, 1.8 °C/s, 1.9 °C/s, 2 °C/s, 2.1 °C/s, 2.2 °C/s, 2.3 °C/s, 2.4 °C/s, 2.5 °C/s, 2.6 °C/s, 2.7 °C/s, 2.8 °C/s, 2.9 °C/s, 3 °C/s, 3.1 °C/s, 3.2 °C/s, 3.3 °C/s, 3.4 °C/s, 3.5 °C/s, 3.6 °C/s, 3.7 °C/s, 3.8 °C/s, 3.9 °C/s, 4 °C/s, 4.1 °C/s, 4.2 °C/s, 4.3 °C/s, 4.4 °C/s, 4.5 °C/s, 4.6 °C/s, 4.7 °C/s, 4.8 °C/s, 4.9 °C/s, 5 °C/s, or a rate between any two of these values.

In some embodiments, after completion of one pulse and before performing a next pulse on the reaction system of S160, the temperature of the reaction system of S160 is restored to the temperature prior to the pulsed heating. As such, hydrogen bonds between GCs in the secondary structure formed in the template molecule can be repeatedly disrupted to completely open the secondary structure in the template molecule, which facilitates the binding of the template molecule to the amplification primer and the extension of the amplification primer, thereby improving amplification efficiency and reducing GC bias.

In addition to the amplification method described above, as shown in FIGs. 1 and 2, the present application also provides another amplification method, which includes:
S200, providing a surface of a solid carrier, which includes a first amplification primer, a second amplification primer, and a target polynucleotide that are immobilized thereon, at least a portion of the target polynucleotide complementarily hybridizing with the first amplification primer.

The surface of the solid carrier may be, for example, a surface of a sequencing chip. Illustratively, the surface of the solid carrier includes a plurality of nanopores arranged in an array. The size of the nanopores and the pore spacing distance may be determined according to actual conditions. The first amplification primer and the second amplification primer are immobilized on the inner surface of the nanopore by surface chemical modification. The first amplification primer and the second amplification primer are, for example, single-stranded oligonucleotides. The first amplification primer and the second amplification primer can be immobilized on the surface of the solid carrier by means known in the art, such as covalent bonding or physical adsorption. The target polynucleotide may be, for example, a single-stranded nucleic acid molecule or a nucleic acid analogue, and at least a portion of one terminus of the target polynucleotide complementarily hybridizes with the first amplification primer in the manner of base complementary pairing to form a hybrid complex, thereby immobilizing the target polynucleotide on the surface of the solid carrier. The first amplification primer that complementarily hybridizes with the target polynucleotide can serve as a starting point for synthesizing the complementary strand of the target polynucleotide, and under the action of a DNA polymerase and conditions suitable for polymerase chain reaction, it initiates the synthesis of the complementary strand of the target polynucleotide.

As shown in FIG. 1 and FIG. 2, the amplification method provided in the embodiments of the present application further includes:
S400, extending the first amplification primer by taking the target polynucleotide as a template and using a first solution or a second solution to obtain an extended strand of the first amplification primer. The first solution or the second solution contains components required for extension, such as DNA polymerases, nucleotides (e.g., A, T, C, G), or nucleotide analogs. Under the action of a DNA polymerase and conditions suitable for polymerase chain reaction, nucleotides or nucleotide analogs are bound to the 3' terminus of the first amplification primer by taking the target polynucleotide as a template, thereby extending the first amplification primer. The obtained extended strand of the first amplification primer is the complementary strand of the target polynucleotide. Illustratively, the DNA polymerase is selected from one or more of rTaq, Canace, Pfu, KOD, Phusion, Primerstar, Tth, BST, and BSU. Preferably, the DNA polymerase has a concentration of 4 U/mL.

As shown in FIG. 1 and FIG. 2, the amplification method provided in the embodiments of the present application further includes:
S600, replacing the first solution or the second solution with a third solution to remove the target polynucleotide, where the third solution contains a denaturing reagent.

By replacing the reacted first solution or second solution with the third solution, the denaturing reagent in the third solution can be used to weaken or disrupt the molecular force between the target polynucleotide and the complementary strand thereof, for example, to break the hydrogen bond between the target polynucleotide and the complementary strand thereof, so that the double-stranded molecules are melted to form single-stranded molecules, thereby removing the target polynucleotide. Illustratively, the denaturing reagent may be, for example, a strong acid (e.g., HCl), a strong base (e.g., NaOH), urea, methanol, ethanol, an amide compound, guanidine hydrochloride, guanidine isothiocyanate, or the like. Preferably, the denaturing reagent is an amide compound, such as formamide.

As shown in FIG. 1 and FIG. 2, the amplification method provided in the embodiments of the present application further includes:
S800, replacing the third solution with a fourth solution to allow the extended strand of the first amplification primer to bind to the second amplification primer.

The free terminus of the newly synthesized extended strand of the first amplification primer includes a portion capable of complementarily hybridizing with the second amplification primer. The fourth solution contains components required for hybridization. Illustratively, the fourth solution may be the first solution or the second solution without the DNA polymerase and the nucleotides or nucleotide analogs. By replacing the third solution with the fourth solution, the free terminus of the extended strand of the first amplification primer can hybridize with the second amplification primer on the surface of the solid carrier, so as to extend the second amplification primer by taking the extended strand of the first amplification primer as a template to obtain the extended strand of the second amplification primer, i.e., the complementary strand of the extended strand of the first amplification primer.

As shown in FIG. 1 and FIG. 2, the amplification method provided in the embodiments of the present application further includes:
S1000, extending the second amplification primer by taking the extended strand of the first amplification primer as a template and replacing the fourth solution with the first solution or the second solution to obtain an extended strand of the second amplification primer.

The first solution or the second solution contains components required for extension, such as DNA polymerases, nucleotides, or nucleotide analogs. Under the action of a DNA polymerase and conditions suitable for polymerase chain reaction, nucleotides or nucleotide analogs are bound to the 3' terminus of the second amplification primer by taking the extended strand of the first amplification primer as a template, thereby extending the second amplification primer. The obtained extended strand of the second amplification primer is the complementary strand of the extended strand of the first amplification primer.

As shown in FIG. 1 and FIG. 2, the amplification method provided in the embodiments of the present application further includes:
S1200, replacing the first solution or the second solution with the third solution.

The third solution contains a denaturing reagent. By replacing the reacted first solution or second solution with the third solution, the denaturing reagent in the third solution can be used to weaken or disrupt the molecular force between the extended strand of the first amplification primer and the extended strand of the second amplification primer, for example, to break the hydrogen bond between the extended strand of the first amplification primer and the extended strand of the second amplification primer, so that the double-stranded molecules are melted to form single-stranded molecules.

The amplification method provided in the embodiments of the present application further includes:
S1400, replacing the third solution with the fourth solution to allow the extended strand of the first amplification primer and the extended strand of the second amplification primer to bind to the second amplification primer and the first amplification primer, respectively.

The free terminus of the extended strand of the first amplification primer includes a portion capable of complementarily hybridizing with the second amplification primer, and the free terminus of the extended strand of the second amplification primer includes a portion capable of complementarily hybridizing with the first amplification primer. The fourth solution contains components required for hybridization. Illustratively, the fourth solution may be the first solution or the second solution without the DNA polymerase and the nucleotides or nucleotide analogs. By replacing the third solution with the fourth solution, the free terminus of the extended strand of the first amplification primer can hybridize with the second amplification primer on the surface of the solid carrier, and the free terminus of the extended strand of the second amplification primer can hybridize with the first amplification primer on the surface of the solid carrier.

The amplification method provided in the embodiments of the present application further includes:
S1600, extending the first amplification primer and the second amplification primer by replacing the fourth solution with the first solution or the second solution and taking the extended strand of the first amplification primer and the extended strand of the second amplification primer as templates.

The first solution or the second solution contains components required for extension, such as DNA polymerases, nucleotides, or nucleotide analogs. Under the action of a DNA polymerase and conditions suitable for polymerase chain reaction:
nucleotides or nucleotide analogs are bound to the 3' terminus of the second amplification primer by taking the extended strand of the first amplification primer as a template to extend the second amplification primer, thereby obtaining an extended strand of the second amplification primer, which is the complementary strand of the extended strand of the first amplification primer, i.e., the target polynucleotide; and
nucleotides or nucleotide analogs are bound to the 3' terminus of the first amplification primer by taking the extended strand of the second amplification primer as a template to extend the first amplification primer, thereby obtaining an extended strand of the first amplification primer, which is the complementary strand of the extended strand of the second amplification primer, i.e., the complementary strand of the target polynucleotide.

The amplification method provided in the embodiments of the present application further includes: S1800, repeating S1200-S1600 at least once.

As such, multiple target polynucleotides can be obtained, i.e., forming a molecular cluster of target polynucleotides.

In the amplification method provided in the present application, a first cycle of amplification is performed on the target polynucleotide through steps S200-S400, and the extended strand of the first amplification primer is generated on the surface of the solid carrier; then, amplification is performed on the extended strand of the first amplification primer through steps S600-S1000, and the extended strand of the second amplification primer is generated on the surface of the solid carrier; subsequently, the extended strand of the first amplification primer and the extended strand of the second amplification primer are used as templates to perform cyclic amplification according to steps S1200-S1600 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection.

In the embodiments of the present application, S1600 is performed under a variable-temperature condition, S1200 and S1400 are performed under a constant-temperature condition, and a reaction temperature of S1600 is not lower than reaction temperatures of S1200 and S1400.

The inventors of the present application have found through research that the temperature can affect the stability of the secondary structure formed due to high GC content in the template molecule, and a higher temperature is more likely to promote melting of the secondary structure in the template molecule. Based on this finding, the present application promotes the melting of the secondary structure formed due to high GC content in the template molecule by introducing variable-temperature amplification during the constant-temperature amplification, thereby facilitating the binding of the template molecule to the amplification primer and the extension of the amplification primer, improving amplification efficiency, and reducing GC bias.

Specifically, S1600 is performed under a variable-temperature condition, including:
in S1600, performing a first heating on a reaction system of S1600 to raise the temperature of the reaction system of S1600 to a first temperature, and maintaining the first temperature for a first preset duration;
after the first preset duration, performing a second heating on the reaction system of S1600 to raise the temperature of the reaction system of S1600 from the first temperature to a second temperature, and maintaining the second temperature for a second preset duration.

Since the temperature at which the polymerase exhibits optimal activity may be different from the temperature at which the secondary structure melts, the use of a single temperature to complete amplification may not give consideration to both the activity of the polymerase and the melting effect of the secondary structure. Therefore, the present application creatively proposes adopting the above gradient heating for the reaction system of S1600, which can balance the activity of the polymerase in the reaction system and the promoting effect on the melting of the secondary structure formed in the template molecule. For example, supposing that the polymerase exhibits the optimal activity at the first temperature, the temperature, however, does not promote the melting of secondary structures. Therefore, it is conceivable to raise the temperature of the reaction system to a second temperature on the basis of the first temperature and maintain the second temperature for a second preset duration after the first preset duration. As such, the polymerase can maintain good activity to continue to exert its functions, while in terms of the secondary structure in the template molecule, the separation of the two strands is promoted, which facilitates the binding of the template molecule to the amplification primer and the extension of the amplification primer, thereby improving amplification efficiency and reducing GC bias. In some embodiments, the first temperature is 60 °C-65 °C, and the first preset duration is 1-10 s; and/or the second temperature is 80 °C-85 °C, and the second preset duration is 1-10 s. Illustratively, the first temperature may be 60 °C, 61 °C, 62 °C, 63 °C, 64 °C, 65 °C, or a temperature between any two of these values. The first preset duration may be 1 s, 2 s, 3 s, 4 s, 5 s, 6 s, 7 s, 8 s, 9 s, 10 s, or a duration between any two of these values. The second temperature may be 80 °C, 81 °C, 82 °C, 83 °C, 84 °C, 85 °C, or a temperature between any two of these values. The second preset duration may be 1 s, 2 s, 3 s, 4 s, 5 s, 6 s, 7 s, 8 s, 9 s, 10 s, or a duration between any two of these values.

In some embodiments, the first heating rate and the second heating rate are 0.1-5.0 °C/s. Illustratively, the first heating rate and the second heating rate may be 0.1 °C/s, 0.2 °C/s, 0.3 °C/s, 0.4 °C/s, 0.5 °C/s, 0.6 °C/s, 0.7 °C/s, 0.8 °C/s, 0.9 °C/s, 1 °C/s, 1.1 °C/s, 1.2 °C/s, 1.3 °C/s, 1.4 °C/s, 1.5 °C/s, 1.6 °C/s, 1.7 °C/s, 1.8 °C/s, 1.9 °C/s, 2 °C/s, 2.1 °C/s, 2.2 °C/s, 2.3 °C/s, 2.4 °C/s, 2.5 °C/s, 2.6 °C/s, 2.7 °C/s, 2.8 °C/s, 2.9 °C/s, 3 °C/s, 3.1 °C/s, 3.2 °C/s, 3.3 °C/s, 3.4 °C/s, 3.5 °C/s, 3.6 °C/s, 3.7 °C/s, 3.8 °C/s, 3.9 °C/s, 4 °C/s, 4.1 °C/s, 4.2 °C/s, 4.3 °C/s, 4.4 °C/s, 4.5 °C/s, 4.6 °C/s, 4.7 °C/s, 4.8 °C/s, 4.9 °C/s, 5 °C/s, or a rate between any two of these values.

In some embodiments, S1600 is performed under a variable-temperature condition, including:
in S1600, performing at least one pulsed heating on the reaction system of S1600 to raise the temperature of the reaction system of S1600 to a third temperature, where the duration of one pulse is a third preset duration.

By adopting pulsed heating, hydrogen bonds between GCs in the secondary structure formed in the template molecule can be more effectively disrupted, so that the secondary structure is more susceptible to melting, which facilitates the binding of the template molecule to the amplification primer and the extension of the amplification primer, thereby improving amplification efficiency and reducing GC bias.

In some embodiments, the third temperature is 60 °C-85 °C, and the third preset duration is 1-10 s. The third temperature may be 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, 75 °C, 76 °C, 77 °C, 78 °C, 79 °C, 80 °C, 81 °C, 82 °C, 83 °C, 84 °C, 85 °C, or a temperature between any two of these values. The third preset duration may be 1 s, 2 s, 3 s, 4 s, 5 s, 6 s, 7 s, 8 s, 9 s, 10 s, or a duration between any two of these values.

In some embodiments, the pulsed heating has a heating rate of 0.1-5.0 °C/s. The pulsed heating may have a heating rate of 0.1 °C/s, 0.2 °C/s, 0.3 °C/s, 0.4 °C/s, 0.5 °C/s, 0.6 °C/s, 0.7 °C/s, 0.8 °C/s, 0.9 °C/s, 1 °C/s, 1.1 °C/s, 1.2 °C/s, 1.3 °C/s, 1.4 °C/s, 1.5 °C/s, 1.6 °C/s, 1.7 °C/s, 1.8 °C/s, 1.9 °C/s, 2 °C/s, 2.1 °C/s, 2.2 °C/s, 2.3 °C/s, 2.4 °C/s, 2.5 °C/s, 2.6 °C/s, 2.7 °C/s, 2.8 °C/s, 2.9 °C/s, 3 °C/s, 3.1 °C/s, 3.2 °C/s, 3.3 °C/s, 3.4 °C/s, 3.5 °C/s, 3.6 °C/s, 3.7 °C/s, 3.8 °C/s, 3.9 °C/s, 4 °C/s, 4.1 °C/s, 4.2 °C/s, 4.3 °C/s, 4.4 °C/s, 4.5 °C/s, 4.6 °C/s, 4.7 °C/s, 4.8 °C/s, 4.9 °C/s, 5 °C/s, or a rate between any two of these values.

In some embodiments, after completion of one pulse and before performing a next pulse on the reaction system of S1600, the temperature of the reaction system of S1600 is restored to the temperature prior to the pulsed heating. As such, hydrogen bonds between GCs in the secondary structure formed in the template molecule can be repeatedly disrupted to completely open the secondary structure in the template molecule, which facilitates the binding of the template molecule to the amplification primer and the extension of the amplification primer, thereby improving amplification efficiency and reducing GC bias.

In the embodiments of the present application, the first solution, the second solution, and the fourth solution all further include at least one of betaine, Mg²⁺, NH₄⁺, and formamide, where the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume (vol/vol% or v/v%) less than or equal to 15%. By configuring at least one of betaine having a concentration greater than 0 mol/L and less than or equal to 4 mol/L, Mg²⁺ having a concentration of 0.5-4 mmol/L, NH₄⁺ having a concentration greater than 0 mmol/L and less than or equal to 100 mmol/L, and formamide having a percent concentration by volume (vol/vol% or v/v%) less than or equal to 15% in each of the first solution, the second solution, and the fourth solution, the amplification method provided in the present application can reduce the formation of a secondary structure in the template molecule, improve amplification efficiency, and facilitate the binding of the template molecule to the amplification primer and the extension of the amplification primer, thereby reducing GC bias.

Specifically, the inventors of the present application have found through research that betaine can reduce the formation of secondary structures in the template molecule due to GC enrichment, improve the amplification efficiency of GC-rich sequences, and thus reduce GC bias. The inventors believe this may be attributed to betaine's ability to increase hydration in guanine- and cytosine-rich regions of the template molecule, influencing the structure of the template molecule and changing its flexibility. This facilitates DNA polymerase extension along the template molecule, thereby reducing the formation of secondary structures in the template molecule, improving amplification efficiency, and reducing GC bias. Furthermore, betaine can lower the melting temperature of sequences rich in GC content, facilitating more uniform denaturation of double-stranded molecules and reducing melting stability variations caused by differences in GC content, thereby reducing GC bias. Additionally, betaine can stabilize template molecule-protein complexes, helping to maintain the stability of template molecules during amplification and reducing polymerase dissociation induced by GC-rich regions. The inventors of the present application have also found through research that betaine exerts the effects described above or produces the outcomes described above within a certain concentration range. If the concentration of betaine exceeds this range, the amplification is inhibited.

In some embodiments, the betaine has a concentration greater than 0 mol/L and less than or equal to 3 mol/L in the first solution. Illustratively, the betaine may have a concentration of 0.5 mol/L, 1 mol/L, 1.5 mol/L, 2 mol/L, 2.5 mol/L, 3 mol/L, or a concentration between any two of these values in the first solution.

In some embodiments, the betaine may have a concentration of 1.5-2.5 mol/L in the second solution. Illustratively, the betaine may have a concentration of 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or a concentration between any two of these values in the second solution.

In some embodiments, the betaine has a concentration of 1.5-2.5 mol/L in the fourth solution. Illustratively, the betaine may have a concentration of 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or a concentration between any two of these values in the fourth solution.

In some embodiments, the Mg²⁺ may have a concentration of 0.5 mmol/L, 1 mmol/L, 1.5 mmol/L, 2 mmol/L, 2.5 mmol/L, 3 mmol/L, 3.5 mmol/L, 4 mmol/L, or a concentration between two of the values described above.

The inventors of the present application have also found through research that Mg²⁺ can be used as an activity additive of the polymerase and is conducive to activating the activity of the polymerase, so that the polymerase catalyzes the formation of a phosphodiester bond between the 3'-OH of the amplification primer bound to the template molecule and the phosphate group of the nucleotide or nucleotide analog, thereby binding the nucleotide or nucleotide analog to the amplification primer and binding the nucleotide or nucleotide analog to the template molecule in the manner of base complementary pairing, that is, the nucleotide or nucleotide analog is incorporated into the template molecule. This improves amplification efficiency and reduces GC bias, thereby helping to minimize the intensity differences in signals generated during sequencing between molecular clusters of target polynucleotides with high AT and high GC and improving the sequencing accuracy. In addition, the concentration of Mg²⁺ is also critical to the amplification. An excessively low concentration of Mg²⁺ can lead to failure of amplification, and an excessively high concentration can lead to increased base mismatches during amplification. Therefore, a suitable Mg²⁺ concentration should be selected.

In some embodiments, in S400, the first amplification primer is extended by using the first solution, the Mg²⁺ has a concentration of 2-4 mmol/L in the first solution, and the betaine has a concentration of 0.1-2 mol/L in the first solution. In the first cycle of amplification, the synergistic use of a higher concentration of Mg²⁺ and a lower concentration of betaine can improve amplification efficiency, reduce GC bias, and facilitate the extension of the first amplification primer to generate the extended strand of the first amplification primer.

In S1000 or S1600, the fourth solution is replaced with the second solution, the Mg²⁺ has a concentration of 2-4 mmol/L in the second solution, and the betaine has a concentration of 0.1-2 mol/L in the second solution; or in S1000 or S1600, the fourth solution is replaced with the second solution, the Mg²⁺ has a concentration of 1-1.5 mmol/L in the second solution, and the betaine has a concentration of 2-3 mol/L in the second solution.

The inventors of the present application have found that during the extension of the first amplification primer and/or the second amplification primer of S1000 or S1600, replacing the fourth solution with the second solution containing 2-4 mmol/L Mg²⁺ and 0.1-2 mol/L betaine, or replacing the fourth solution with the second solution containing 1-1.5 mmol/L Mg²⁺ and 2-3 mol/L betaine can effectively extend the first amplification primer and/or the second amplification primer, thereby generating the extended strand of the first amplification primer and/or the extended strand of the second amplification primer. The inventors speculate that this may be related to the improved amplification efficiency and reduced GC bias achieved in S400 through the synergistic use of a higher concentration of Mg²⁺ and a lower concentration of betaine, thereby providing more feasible schemes for subsequent amplification steps. In some embodiments, in S400, the first amplification primer is extended by using the second solution, the Mg²⁺ has a concentration of 2-4 mmol/L in the second solution, and the betaine has a concentration of 0.1-2 mol/L in the second solution. In the first cycle of amplification, the synergistic use of a higher concentration of Mg²⁺ and a lower concentration of betaine can improve amplification efficiency, reduce GC bias, and facilitate the extension of the first amplification primer to generate the extended strand of the first amplification primer.

In S1000 or S1600, the fourth solution is replaced with the first solution, the Mg²⁺ has a concentration of 2-4 mmol/L in the first solution, and the betaine has a concentration of 0.1-2 mol/L in the first solution; or in S1000 or S1600, the fourth solution is replaced with the first solution, the Mg²⁺ has a concentration of 1-1.5 mmol/L in the first solution, and the betaine has a concentration of 2-3 mol/L in the first solution. The inventors of the present application have found that during the extension of the first amplification primer and/or the second amplification primer of S1000 or S1600, replacing the fourth solution with the first solution containing 2-4 mmol/L Mg²⁺ and 0.1-2 mol/L betaine, or replacing the fourth solution with the first solution containing 1-1.5 mmol/L Mg²⁺ and 2-3 mol/L betaine can effectively extend the first amplification primer and/or the second amplification primer, thereby generating the extended strand of the first amplification primer and/or the extended strand of the second amplification primer. The inventors speculate that this may be related to the improved amplification efficiency and reduced GC bias achieved in S40 through the synergistic use of a higher concentration of Mg²⁺ and a lower concentration of betaine, thereby providing more feasible schemes for subsequent amplification steps.

In some embodiments, in S400, the first amplification primer is extended by using the first solution, the Mg²⁺ has a concentration of 2-4 mmol/L in the first solution, and the betaine has a concentration of 0.1-2 mol/L in the first solution; in S1000 or S1600, the fourth solution is replaced with the second solution, the Mg²⁺ has a concentration of 2-4 mmol/L in the second solution, and the betaine has a concentration of 0.1-2 mol/L in the second solution; or
in S400, the first amplification primer is extended by using the second solution, the Mg²⁺ has a concentration of 2-4 mmol/L in the second solution, and the betaine has a concentration of 0.1-2 mol/L in the second solution; in S1000 or S1600, the fourth solution is replaced with the first solution, the Mg²⁺ has a concentration of 2-4 mmol/L in the first solution, and the betaine has a concentration of 0.1-2 mol/L in the first solution;
the first solution or the second solution further includes dNTPs, the dNTPs including dATP, dTTP, dCTP, and dGTP, and a sum of contents of dATP and dTTP being equal to a sum of contents of dGTP and dCTP. The amplification primer is extended by using the first solution or the second solution. Particularly, in S40, a base solution with a balanced ratio, i.e., the sum of the contents of dATP and dTTP being equal to the sum of the contents of dGTP and dCTP, is configured in the first solution or the second solution, and this facilitates the amplification of all target polynucleotide molecules as much as possible. Illustratively, dATP, dTTP, dCTP, and dGTP may be present at equal and balanced concentrations; for example, each of dATP, dTTP, dCTP, and dGTP may have a concentration of 200 µmol/L, 100 µmol/L, 50 µmol/L, 10 µmol/L, 5 µmol/L, 1 µmol/L, or the like.

In some embodiments, in S400, the first amplification primer is extended by using the first solution, the Mg²⁺ has a concentration of 2-4 mmol/L in the first solution, and the betaine has a concentration of 0.1-2 mol/L in the first solution; in S1000 or S1600, the fourth solution is replaced with the second solution, the Mg²⁺ has a concentration of 1-1.5 mmol/L in the second solution, and the betaine has a concentration of 2-3 mol/L in the second solution; or
in S400, the first amplification primer is extended by using the second solution, the Mg²⁺ has a concentration of 2-4 mmol/L in the second solution, and the betaine has a concentration of 0.1-2 mol/L in the second solution; in S1000 or S1600, the fourth solution is replaced with the first solution, the Mg²⁺ has a concentration of 1-1.5 mmol/L in the first solution, and the betaine has a concentration of 2-3 mol/L in the first solution;
the second solution or the first solution further includes dNTPs, the dNTPs including dATP, dTTP, dCTP, and dGTP, and a sum of contents of the dATP and the dTTP being less than a sum of contents of the dGTP and the dCTP. The amplification primer is extended by using the first solution or the second solution, and particularly, in S1000 or S1600, preparing a base solution with an imbalanced ratio, i.e., a sum of the contents of dATP and dTTP being less than a sum of the contents of dGTP and dCTP, in the first solution or the second solution is beneficial to increasing the probability of complementary pairing of dGTP and dCTP with GC bases on the template molecule and preferentially amplifying sequences with a high GC content, thereby enhancing the amplification of GC bases in the template molecule, improving amplification efficiency, and reducing GC bias. Illustratively, the sum of the contents of dATP and dTTP is 90%, 80%, 70%, 60%, 50%, 40%, 30%, 20%, 10%, 5%, or the like of the sum of the contents of dGTP and dCTP.

The inventors of the present application have also found through research that NH₄⁺ can improve the reactivity of the polymerase, thereby improving amplification efficiency and reducing GC bias. The inventors speculate that the increase in the reactivity of the polymerase by NH₄⁺ may be associated with the ability of NH₄⁺ to change the charge distribution on the surface of the polymerase and/or to change the spatial configuration of the polymerase, such that the polymerase can be more easily bound to the reaction sites of the template molecule and/or the amplification primer and catalyzes the formation of a phosphodiester bond between the 3'-OH of the amplification primer and the phosphate group of the nucleotide or nucleotide analog, thereby binding the nucleotide or nucleotide analog to the amplification primer and binding the nucleotide or nucleotide analog to the nucleic acid template via complementary base pairing, i.e., incorporating the nucleotide or nucleotide analog into the template molecule.

In some embodiments, the NH₄⁺ may have a concentration of 0.1 mmol/L, 1 mmol/L, 5 mmol/L, 10 mmol/L, 20 mmol/L, 30 mmol/L, 40 mmol/L, 50 mmol/L, 60 mmol/L, 70 mmol/L, 80 mmol/L, 90 mmol/L, 100 mmol/L, or a concentration between any two of these values.

In some embodiments, the NH₄⁺ is NH₄⁺ provided by at least one of ammonium sulfate, ammonium chloride, and ammonium acetate. That is, the first solution, the second solution, and the fourth solution of the present application all include at least one of ammonium sulfate, ammonium chloride, and ammonium acetate, or at least one of ammonium sulfate, ammonium chloride, and ammonium acetate is added when the first solution, the second solution, and the fourth solution are prepared.

In addition, the inventors of the present application have also found that formamide can be used as a denaturing reagent, and can reduce the stability of double-stranded molecules in the secondary structure in the template molecule, disrupt the hydrogen bonds between the double-stranded molecules, cause melting of the double-stranded molecules at a relatively low temperature, and facilitate the binding of the template molecule to the amplification primer, which facilitates the amplification, thereby improving amplification efficiency and reducing GC bias. This difference in effect is mainly related to the concentration of formamide in the solution. For example, when the percent concentration by volume (vol/vol% or v/v%) of formamide reaches or exceeds 60%, e.g., 60-100%, formamide is used as a denaturing reagent, and when the percent concentration by volume (vol/vol% or v/v%) of formamide is greater than or equal to 3% and less than or equal to 15%, formamide can reduce the stability of double-stranded molecules in the secondary structure in the template molecule, disrupt the hydrogen bonds between the double-stranded molecules, cause melting of the double-stranded molecules at a relatively low temperature, and facilitate the binding of the template molecule to the amplification primer, which facilitates the amplification, thereby improving amplification efficiency and reducing GC bias. Therefore, by controlling the concentration of formamide, the formamide can be controlled to exert different effects and achieve different purposes.

In some embodiments, the formamide may have a percent concentration by volume (vol/vol% or v/v%) of 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, or a percent concentration by volume (vol/vol% or v/v%) between any two of these values in the first solution, the second solution, and the fourth solution.

In the embodiments of the present application, the first solution, the second solution, and the fourth solution all include DMSO (dimethyl sulfoxide), and the DMSO has a percent concentration by volume (vol/vol% or v/v%) of 1-5% in each of the first solution, the second solution, and the fourth solution.

Since sequences with a high GC content in the template molecule are often difficult to amplify due to the easy formation of secondary structures, DMSO can improve the spatial configuration of the template molecule with a high GC content, reduce the formation of secondary structures, and enable the polymerase to extend along the template molecule, thereby improving amplification efficiency and reducing GC bias.

In some embodiments, the DMSO may have a percent concentration by volume (vol/vol% or v/v%) of 1%, 1.5%, 2%, 2.5%, 3%, 3.5%, 4%, 4.5%, 5%, or a concentration between any two of these values in each of the first solution, the second solution, and the fourth solution.

In the embodiments of the present application, the first solution, the second solution, and the fourth solution further include Trisbase (tris(hydroxymethyl)aminomethane), and the Trisbase has a concentration of 10-500 mmol/L in each of the first solution, the second solution, and the fourth solution. The Trisbase can provide an appropriate buffer environment and ionic strength for amplification.

In some embodiments, the Trisbase may have a concentration of 10 mmol/L, 15 mmol/L, 20 mmol/L, 25 mmol/L, 30 mmol/L, 35 mmol/L, 40 mmol/L, 45 mmol/L, 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, 200 mmol/L, 210 mmol/L, 220 mmol/L, 230 mmol/L, 240 mmol/L, 250 mmol/L, 260 mmol/L, 270 mmol/L, 280 mmol/L, 290 mmol/L, 300 mmol/L, 310 mmol/L, 320 mmol/L, 330 mmol/L, 340 mmol/L, 350 mmol/L, 360 mmol/L, 370 mmol/L, 380 mmol/L, 390 mmol/L, 400 mmol/L, 410 mmol/L, 420 mmol/L, 430 mmol/L, 440 mmol/L, 450 mmol/L, 460 mmol/L, 470 mmol/L, 480 mmol/L, 490 mmol/L, 500 mmol/L, or a concentration between any two of these values in the first solution, the second solution, and the fourth solution.

In the embodiments of the present application, the first solution, the second solution, and the fourth solution further include a single-stranded binding protein and/or bovine serum albumin. The single-stranded binding protein and/or bovine serum albumin can protect the DNA polymerase and effectively promote the amplification of the template molecule and reduce GC bias. The single-stranded binding protein has a concentration of 0.01-1 mg/mL in the first solution, the second solution, and the fourth solution; the bovine serum albumin has a percent concentration by volume (vol/vol% or v/v%) of 0.05-2% in the first solution, the second solution, and the fourth solution. Illustratively, the single-stranded binding protein may have a concentration of 0.01 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.7 mg/mL, 0.75 mg/mL, 0.8 mg/mL, 0.85 mg/mL, 0.9 mg/mL, 0.95 mg/mL, 1 mg/mL, or a concentration between any two of these values in the first solution, the second solution, and the fourth solution. The bovine serum albumin may have a percent concentration by volume (vol/vol% or v/v%) of 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.05%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, or a concentration between any two of these values in the first solution, the second solution, and the fourth solution.

In the embodiments of the present application, the first solution, the second solution, and the fourth solution further include 10-50 mmol/L tetramethylammonium chloride or tetraethylammonium chloride. Although the inventors are not aware of the reason, through research, tetramethylammonium chloride or tetraethylammonium chloride is found to be able to facilitate the amplification of the template molecule, thereby reducing GC bias. Illustratively, the tetramethylammonium chloride or tetraethylammonium chloride may have a concentration of 10 mmol/L, 20 mmol/L, 30 mmol/L, 40 mmol/L, 50 mmol/L, 60 mmol/L, or a concentration between any two of these values.

In the embodiments of the present application, the first solution, the second solution, and the fourth solution further include a surfactant having a percent concentration by volume (vol/vol% or v/v%) of 0.01-2%. Illustratively, the surfactant is a nonionic surfactant, for example, selected from one or more of Tween 20, DD (N,N-dimethyldodecylamine N-oxide), DDM (n-dodecyl-β-D-maltoside), and Triton X-100 (polyethylene glycol octylphenyl ether).

In some embodiments, the surfactant may have a concentration of 0.01%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.05%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, or a concentration between any two of these values.

In addition to the two amplification methods described above, as shown in FIGs. 1 and 2, the embodiments of the present application also provide a third amplification method, which includes: S2000, providing a surface of a solid carrier, which includes a first amplification primer, a second amplification primer, and a target polynucleotide that are immobilized thereon, at least a portion of the target polynucleotide complementarily hybridizing with the first amplification primer.

The surface of the solid carrier may be, for example, a surface of a sequencing chip. Illustratively, the surface of the solid carrier includes a plurality of nanopores arranged in an array. The size of the nanopores and the pore spacing distance may be determined according to actual conditions. The first amplification primer and the second amplification primer are immobilized on the inner surface of the nanopore by surface chemical modification. The first amplification primer and the second amplification primer are, for example, single-stranded oligonucleotides. The first amplification primer and the second amplification primer can be immobilized on the surface of the solid carrier by means known in the art, such as covalent bonding or physical adsorption. The target polynucleotide may be, for example, a single-stranded nucleic acid molecule or a nucleic acid analogue, and at least a portion of one terminus of the target polynucleotide complementarily hybridizes with the first amplification primer in the manner of base complementary pairing to form a hybrid complex, thereby immobilizing the target polynucleotide on the surface of the solid carrier. The first amplification primer that complementarily hybridizes with the target polynucleotide can serve as a starting point for synthesizing the complementary strand of the target polynucleotide, and under the action of a DNA polymerase and conditions suitable for polymerase chain reaction, it initiates the synthesis of the complementary strand of the target polynucleotide.

As shown in FIG. 1 and FIG. 2, the amplification method provided in the embodiments of the present application further includes:
S4000, extending the first amplification primer by taking the target polynucleotide as a template and using a first solution or a second solution to obtain an extended strand of the first amplification primer. The first solution or the second solution contains components required for extension, such as DNA polymerases, nucleotides (e.g., A, T, C, G), or nucleotide analogs. Under the action of a DNA polymerase and conditions suitable for polymerase chain reaction, nucleotides or nucleotide analogs are bound to the 3' terminus of the first amplification primer by taking the target polynucleotide as a template, thereby extending the first amplification primer. The obtained extended strand of the first amplification primer is the complementary strand of the target polynucleotide. Illustratively, the DNA polymerase is selected from one or more of rTaq, Canace, Pfu, KOD, Phusion, Primerstar, Tth, BST, and BSU. Preferably, the DNA polymerase has a concentration of 4 U/mL.

As shown in FIG. 1 and FIG. 2, the amplification method provided in the embodiments of the present application further includes:
S6000, replacing the first solution or the second solution with a third solution to remove the target polynucleotide, where the third solution contains a denaturing reagent.

By replacing the reacted first solution or second solution with the third solution, the denaturing reagent in the third solution can be used to weaken or disrupt the molecular force between the target polynucleotide and the complementary strand thereof, for example, to break the hydrogen bond between the target polynucleotide and the complementary strand thereof, so that the double-stranded molecules are melted to form single-stranded molecules, thereby removing the target polynucleotide. Illustratively, the denaturing reagent may be, for example, a strong acid (e.g., HCl), a strong base (e.g., NaOH), urea, methanol, ethanol, an amide compound, guanidine hydrochloride, guanidine isothiocyanate, or the like. Preferably, the denaturing reagent is an amide compound, such as formamide.

As shown in FIG. 1 and FIG. 2, the amplification method provided in the embodiments of the present application further includes:
S8000, replacing the third solution with a fourth solution to allow the extended strand of the first amplification primer to bind to the second amplification primer.

The free terminus of the newly synthesized extended strand of the first amplification primer includes a portion capable of complementarily hybridizing with the second amplification primer. The fourth solution contains components required for hybridization. Illustratively, the fourth solution may be the first solution or the second solution without the DNA polymerase and the nucleotides or nucleotide analogs. By replacing the third solution with the fourth solution, the free terminus of the extended strand of the first amplification primer can hybridize with the second amplification primer on the surface of the solid carrier, so as to extend the second amplification primer by taking the extended strand of the first amplification primer as a template to obtain the extended strand of the second amplification primer, i.e., the complementary strand of the extended strand of the first amplification primer.

As shown in FIG. 1 and FIG. 2, the amplification method provided in the embodiments of the present application further includes:
S10000, extending the second amplification primer by taking the extended strand of the first amplification primer as a template and replacing the fourth solution with the first solution or the second solution to obtain an extended strand of the second amplification primer.

The first solution or the second solution contains components required for extension, such as DNA polymerases, nucleotides, or nucleotide analogs. Under the action of a DNA polymerase and conditions suitable for polymerase chain reaction, nucleotides or nucleotide analogs are bound to the 3' terminus of the second amplification primer by taking the extended strand of the first amplification primer as a template, thereby extending the second amplification primer. The obtained extended strand of the second amplification primer is the complementary strand of the extended strand of the first amplification primer. As shown in FIG. 1 and FIG. 2, the amplification method provided in the embodiments of the present application further includes:
S12000, replacing the first solution or the second solution with the third solution.

The third solution contains a denaturing reagent. By replacing the reacted first solution or second solution with the third solution, the denaturing reagent in the third solution can be used to weaken or disrupt the molecular force between the extended strand of the first amplification primer and the extended strand of the second amplification primer, for example, to break the hydrogen bond between the extended strand of the first amplification primer and the extended strand of the second amplification primer, so that the double-stranded molecules are melted to form single-stranded molecules.

The amplification method provided in the embodiments of the present application further includes:
S14000, replacing the third solution with the fourth solution to allow the extended strand of the first amplification primer and the extended strand of the second amplification primer to bind to the second amplification primer and the first amplification primer, respectively.

The free terminus of the extended strand of the first amplification primer includes a portion capable of complementarily hybridizing with the second amplification primer, and the free terminus of the extended strand of the second amplification primer includes a portion capable of complementarily hybridizing with the first amplification primer. The fourth solution contains components required for hybridization. Illustratively, the fourth solution may be the first solution or the second solution without the DNA polymerase and the nucleotides or nucleotide analogs. By replacing the third solution with the fourth solution, the free terminus of the extended strand of the first amplification primer can hybridize with the second amplification primer on the surface of the solid carrier, and the free terminus of the extended strand of the second amplification primer can hybridize with the first amplification primer on the surface of the solid carrier.

The amplification method provided in the embodiments of the present application further includes:
S16000, extending the first amplification primer and the second amplification primer by replacing the fourth solution with the first solution or the second solution and taking the extended strand of the first amplification primer and the extended strand of the second amplification primer as templates.

The first solution or the second solution contains components required for extension, such as DNA polymerases, nucleotides, or nucleotide analogs. Under the action of a DNA polymerase and conditions suitable for polymerase chain reaction:
nucleotides or nucleotide analogs are bound to the 3' terminus of the second amplification primer by taking the extended strand of the first amplification primer as a template to extend the second amplification primer, thereby obtaining an extended strand of the second amplification primer, which is the complementary strand of the extended strand of the first amplification primer, i.e., the target polynucleotide; and
nucleotides or nucleotide analogs are bound to the 3' terminus of the first amplification primer by taking the extended strand of the second amplification primer as a template to extend the first amplification primer, thereby obtaining an extended strand of the first amplification primer, which is the complementary strand of the extended strand of the second amplification primer, i.e., the complementary strand of the target polynucleotide.

The amplification method provided in the embodiments of the present application further includes:
S18000, repeating S12000-S16000 at least once.

As such, multiple target polynucleotides can be obtained, i.e., forming a molecular cluster of target polynucleotides.

In the amplification method provided in the present application, a first cycle of amplification is performed on the target polynucleotide through steps S2000-S4000, and the extended strand of the first amplification primer is generated on the surface of the solid carrier; then, amplification is performed on the extended strand of the first amplification primer through steps S6000-S 10000, and the extended strand of the second amplification primer is generated on the surface of the solid carrier; subsequently, the extended strand of the first amplification primer and the extended strand of the second amplification primer are used as templates to perform cyclic amplification according to steps S12000-S16000 to obtain a molecular cluster of the target polynucleotides, so as to amplify signals generated by incorporating nucleotides or nucleotide analogs into the target polynucleotides in the process of sequencing the target polynucleotides, thereby making it easier to detect the signals and improving the efficiency and accuracy of signal detection.

In the embodiments of the present application, in S10000 and S16000, the first solution or the second solution includes dATP, dTTP, dCTP, and dGTP, and a sum of contents of the dATP and the dTTP is less than a sum of contents of the dGTP and the dCTP.

The amplification primer is extended by using the first solution or the second solution, and particularly, in S10000 or S16000, preparing a base solution with an imbalanced ratio, i.e., a sum of the contents of dATP and dTTP being less than a sum of the contents of dGTP and dCTP, in the first solution or the second solution is beneficial to increasing the probability of complementary pairing of dGTP and dCTP with GC bases on the template molecule and preferentially amplifying sequences with a high GC content, thereby enhancing the amplification of GC bases in the template molecule, improving amplification efficiency, and reducing GC bias. Illustratively, the sum of the contents of dATP and dTTP is 90%, 80%, 70%, 60%, 50%, 40%, 30%, 20%, 10%, 5%, or the like of the sum of the contents of dGTP and dCTP. In the embodiments of the present application, the first solution, the second solution, and the fourth solution all further include at least one of betaine, Mg²⁺, NH₄⁺, and formamide, where the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume (vol/vol% or v/v%) less than or equal to 15%. By configuring at least one of betaine having a concentration greater than 0 mol/L and less than or equal to 4 mol/L, Mg²⁺ having a concentration of 0.5-4 mmol/L, NH₄⁺ having a concentration greater than 0 mmol/L and less than or equal to 100 mmol/L, and formamide having a percent concentration by volume (vol/vol% or v/v%) less than or equal to 15% in each of the first solution, the second solution, and the fourth solution, the amplification method provided in the present application can reduce the formation of a secondary structure in the template molecule, improve amplification efficiency, and facilitate the binding of the template molecule to the amplification primer and the extension of the amplification primer, thereby reducing GC bias.

The inventors of the present application have found through research that betaine can reduce the formation of secondary structures in the template molecule due to GC enrichment, improve the amplification efficiency of GC-rich sequences, and thus reduce GC bias. The inventors believe this may be attributed to betaine's ability to increase hydration in guanine- and cytosine-rich regions of the template molecule, influencing the structure of the template molecule and changing its flexibility. This facilitates DNA polymerase extension along the template molecule, thereby reducing the formation of secondary structures in the template molecule, improving amplification efficiency, and reducing GC bias. Furthermore, betaine can lower the melting temperature of sequences rich in GC content, facilitating more uniform denaturation of double-stranded molecules and reducing melting stability variations caused by differences in GC content, thereby reducing GC bias. Additionally, betaine can stabilize template molecule-protein complexes, helping to maintain the stability of template molecules during amplification and reducing polymerase dissociation induced by GC-rich regions. The inventors of the present application have also found through research that betaine exerts the effects described above or produces the outcomes described above within a certain concentration range. If the concentration of betaine exceeds this range, the amplification is inhibited.

Mg²⁺ can be used as an activity additive of the polymerase and is conducive to activating the activity of the polymerase, so that the polymerase catalyzes the formation of a phosphodiester bond between the 3'-OH of the amplification primer bound to the template molecule and the phosphate group of the nucleotide or nucleotide analog, thereby binding the nucleotide or nucleotide analog to the amplification primer and binding the nucleotide or nucleotide analog to the template molecule in the manner of base complementary pairing, that is, the nucleotide or nucleotide analog is incorporated into the template molecule. This improves amplification efficiency and reduces GC bias, thereby helping to minimize the intensity differences in signals generated during sequencing between molecular clusters of target polynucleotides with high AT and high GC and improving the sequencing accuracy. In addition, the concentration of Mg²⁺ is also critical to the amplification. An excessively low concentration of Mg²⁺ can lead to failure of amplification, and an excessively high concentration can lead to increased base mismatches during amplification. Therefore, a suitable Mg²⁺ concentration should be selected.

In some embodiments, in S4000, the first amplification primer is extended by using the first solution, and in S10000 and S16000, the fourth solution is replaced with the first solution or the second solution, where in the first solution, the Mg²⁺ has a concentration of 2-4 mmol/L, and the betaine has a concentration of 0.1-2 mol/L, and in the second solution, the Mg²⁺ has a concentration of 2-4 mmol/L, and the betaine has a concentration of 0.1-2 mol/L. Alternatively, in S4000, the first amplification primer is extended by using the first solution, and in S10000 and S16000, the fourth solution is replaced with the first solution or the second solution, where in the first solution, the Mg²⁺ has a concentration of 2-4 mmol/L, and the betaine has a concentration of 0.1-2 mol/L, and in the second solution, the Mg²⁺ has a concentration of 1-1.5 mmol/L, and the betaine has a concentration of 2-3 mol/L.

In S4000, the first amplification primer is extended by using the first solution, the Mg²⁺ has a concentration of 2-4 mmol/L in the first solution, and the betaine has a concentration of 0.1-2 mol/L in the first solution. In the first cycle of amplification, the synergistic use of a higher concentration of Mg²⁺ and a lower concentration of betaine can improve amplification efficiency, reduce GC bias, and facilitate the extension of the first amplification primer to generate the extended strand of the first amplification primer. During the extension of the first amplification primer and/or the second amplification primer of S10000 or S16000, replacing the fourth solution with the first solution or the second solution containing 2-4 mmol/L Mg²⁺ and 0.1-2 mol/L betaine, or replacing the fourth solution with the first solution or the second solution containing 1-1.5 mmol/L Mg²⁺ and 2-3 mol/L betaine can effectively extend the first amplification primer and/or the second amplification primer, thereby generating the extended strand of the first amplification primer and/or the extended strand of the second amplification primer. The inventors speculate that this may be related to the improved amplification efficiency and reduced GC bias achieved in the first cycle of amplification through the synergistic use of a higher concentration of Mg²⁺ and a lower concentration of betaine, thereby providing more feasible schemes for subsequent amplification steps.

In some embodiments, in S4000, the first amplification primer is extended by using the second solution, the Mg²⁺ has a concentration of 2-4 mmol/L in the second solution, and the betaine has a concentration of 0.1-2 mol/L in the second solution. In the first cycle of amplification, the synergistic use of a higher concentration of Mg²⁺ and a lower concentration of betaine can improve amplification efficiency, reduce GC bias, and facilitate the extension of the first amplification primer to generate the extended strand of the first amplification primer.

In S10000 or S16000, the fourth solution is replaced with the first solution or the second solution, the Mg²⁺ has a concentration of 2-4 mmol/L in the first solution or the second solution, and the betaine has a concentration of 0.1-2 mol/L in the first solution or the second solution; or in S10000 or S16000, the fourth solution is replaced with the first solution or the second solution, the Mg²⁺ has a concentration of 1-1.5 mmol/L in the first solution or the second solution, and the betaine has a concentration of 2-3 mol/L in the first solution or the second solution.

The inventors of the present application have found that during the extension of the first amplification primer and/or the second amplification primer of S10000 or S16000, replacing the fourth solution with the first solution or the second solution containing 2-4 mmol/L Mg²⁺ and 0.1-2 mol/L betaine, or replacing the fourth solution with the first solution or the second solution containing 1-1.5 mmol/L Mg²⁺ and 2-3 mol/L betaine can effectively extend the first amplification primer and/or the second amplification primer, thereby generating the extended strand of the first amplification primer and/or the extended strand of the second amplification primer. The inventors speculate that this may be related to the improved amplification efficiency and reduced GC bias achieved in the first cycle of amplification through the synergistic use of a higher concentration of Mg²⁺ and a lower concentration of betaine, thereby providing more feasible schemes for subsequent amplification steps.

In some embodiments, in S4000, the first solution or the second solution includes dATP, dTTP, dCTP, and dGTP, and a sum of the contents of the dATP and the dTTP is equal to a sum of the contents of the dGTP and the dCTP.

The amplification primer is extended by using the first solution or the second solution. Particularly, in S4000, a base solution with a balanced ratio, i.e., the sum of the contents of dATP and dTTP being equal to the sum of the contents of dGTP and dCTP, is configured in the first solution or the second solution, and this facilitates the amplification of all target polynucleotide molecules as much as possible. Illustratively, dATP, dTTP, dCTP, and dGTP may be present at equal and balanced concentrations; for example, each of dATP, dTTP, dCTP, and dGTP may have a concentration of 200 µmol/L, 100 µmol/L, 50 µmol/L, 10 µmol/L, 5 µmol/L, 1 µmol/L, or the like.

In some embodiments, the betaine has a concentration of 1.5-2.5 mol/L in the fourth solution.

Illustratively, the betaine may have a concentration of 1.5 mol/L, 1.6 mol/L, 1.7 mol/L, 1.8 mol/L, 1.9 mol/L, 2.0 mol/L, 2.1 mol/L, 2.2 mol/L, 2.3 mol/L, 2.4 mol/L, 2.5 mol/L, or a concentration between any two of these values in the fourth solution.

The inventors of the present application have also found through research that NH₄⁺ can improve the reactivity of the polymerase, thereby improving amplification efficiency and reducing GC bias. The inventors speculate that the increase in the reactivity of the polymerase by NH₄⁺ may be associated with the ability of NH₄⁺ to change the charge distribution on the surface of the polymerase and/or to change the spatial configuration of the polymerase, such that the polymerase can be more easily bound to the reaction sites of the template molecule and/or the amplification primer and catalyzes the formation of a phosphodiester bond between the 3'-OH of the amplification primer and the phosphate group of the nucleotide or nucleotide analog, thereby binding the nucleotide or nucleotide analog to the amplification primer and binding the nucleotide or nucleotide analog to the nucleic acid template via complementary base pairing, i.e., incorporating the nucleotide or nucleotide analog into the template molecule.

In some embodiments, the NH₄⁺ may have a concentration of 0.1 mmol/L, 1 mmol/L, 5 mmol/L, 10 mmol/L, 20 mmol/L, 30 mmol/L, 40 mmol/L, 50 mmol/L, 60 mmol/L, 70 mmol/L, 80 mmol/L, 90 mmol/L, 100 mmol/L, or a concentration between any two of these values.

In some embodiments, the NH₄⁺ is NH₄⁺ provided by at least one of ammonium sulfate, ammonium chloride, and ammonium acetate. That is, the first solution, the second solution, and the fourth solution of the present application all include at least one of ammonium sulfate, ammonium chloride, and ammonium acetate, or at least one of ammonium sulfate, ammonium chloride, and ammonium acetate is added when the first solution, the second solution, and the fourth solution are prepared.

In addition, the inventors of the present application have also found that formamide can be used as a denaturing reagent, and can reduce the stability of double-stranded molecules in the secondary structure in the template molecule, disrupt the hydrogen bonds between the double-stranded molecules, cause melting of the double-stranded molecules at a relatively low temperature, and facilitate the binding of the template molecule to the amplification primer, which facilitates the amplification, thereby improving amplification efficiency and reducing GC bias. This difference in effect is mainly related to the concentration of formamide in the solution. For example, when the percent concentration by volume (vol/vol% or v/v%) of formamide reaches or exceeds 60%, e.g., 60-100%, formamide is used as a denaturing reagent, and when the percent concentration by volume (vol/vol% or v/v%) of formamide is greater than or equal to 3% and less than or equal to 15%, formamide can reduce the stability of double-stranded molecules in the secondary structure in the template molecule, disrupt the hydrogen bonds between the double-stranded molecules, cause melting of the double-stranded molecules at a relatively low temperature, and facilitate the binding of the template molecule to the amplification primer, which facilitates the amplification, thereby improving amplification efficiency and reducing GC bias. Therefore, by controlling the concentration of formamide, the formamide can be controlled to exert different effects and achieve different purposes.

In some embodiments, the formamide may have a percent concentration by volume (vol/vol% or v/v%) of 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, or a percent concentration by volume (vol/vol% or v/v%) between any two of these values in the first solution, the second solution, and the fourth solution.

In the embodiments of the present application, the first solution, the second solution, and the fourth solution all include DMSO (dimethyl sulfoxide), and the DMSO has a percent concentration by volume (vol/vol% or v/v%) of 1-5% in each of the first solution, the second solution, and the fourth solution.

Since sequences with a high GC content in the template molecule are often difficult to amplify due to the easy formation of secondary structures, DMSO can improve the spatial configuration of the template molecule with a high GC content, reduce the formation of secondary structures, and enable the polymerase to extend along the template molecule, thereby improving amplification efficiency and reducing GC bias.

In some embodiments, the DMSO may have a percent concentration by volume (vol/vol% or v/v%) of 1%, 1.5%, 2%, 2.5%, 3%, 3.5%, 4%, 4.5%, 5%, or a concentration between any two of these values in each of the first solution, the second solution, and the fourth solution.

In the embodiments of the present application, the first solution, the second solution, and the fourth solution further include Trisbase (tris(hydroxymethyl)aminomethane), and the Trisbase has a concentration of 10-500 mmol/L in each of the first solution, the second solution, and the fourth solution. The Trisbase can provide an appropriate buffer environment and ionic strength for amplification.

In some embodiments, the Trisbase may have a concentration of 10 mmol/L, 15 mmol/L, 20 mmol/L, 25 mmol/L, 30 mmol/L, 35 mmol/L, 40 mmol/L, 45 mmol/L, 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, 200 mmol/L, 210 mmol/L, 220 mmol/L, 230 mmol/L, 240 mmol/L, 250 mmol/L, 260 mmol/L, 270 mmol/L, 280 mmol/L, 290 mmol/L, 300 mmol/L, 310 mmol/L, 320 mmol/L, 330 mmol/L, 340 mmol/L, 350 mmol/L, 360 mmol/L, 370 mmol/L, 380 mmol/L, 390 mmol/L, 400 mmol/L, 410 mmol/L, 420 mmol/L, 430 mmol/L, 440 mmol/L, 450 mmol/L, 460 mmol/L, 470 mmol/L, 480 mmol/L, 490 mmol/L, 500 mmol/L, or a concentration between any two of these values in the first solution, the second solution, and the fourth solution.

In the embodiments of the present application, the first solution, the second solution, and the fourth solution further include a single-stranded binding protein and/or bovine serum albumin. The single-stranded binding protein and/or bovine serum albumin can protect the DNA polymerase and effectively promote the amplification of the template molecule and reduce GC bias. The single-stranded binding protein has a concentration of 0.01-1 mg/mL in the first solution, the second solution, and the fourth solution; the bovine serum albumin has a percent concentration by volume (vol/vol% or v/v%) of 0.05-2% in the first solution, the second solution, and the fourth solution. Illustratively, the single-stranded binding protein may have a concentration of 0.01 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.7 mg/mL, 0.75 mg/mL, 0.8 mg/mL, 0.85 mg/mL, 0.9 mg/mL, 0.95 mg/mL, 1 mg/mL, or a concentration between any two of these values in the first solution, the second solution, and the fourth solution. The bovine serum albumin may have a percent concentration by volume (vol/vol% or v/v%) of 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.05%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, or a concentration between any two of these values in the first solution, the second solution, and the fourth solution.

In the embodiments of the present application, the first solution, the second solution, and the fourth solution further include 10-50 mmol/L tetramethylammonium chloride or tetraethylammonium chloride. Although the inventors are not aware of the reason, through research, tetramethylammonium chloride or tetraethylammonium chloride is found to be able to facilitate the amplification of the template molecule, thereby reducing GC bias. Illustratively, the tetramethylammonium chloride or tetraethylammonium chloride may have a concentration of 10 mmol/L, 20 mmol/L, 30 mmol/L, 40 mmol/L, 50 mmol/L, 60 mmol/L, or a concentration between any two of these values.

In the embodiments of the present application, the first solution, the second solution, and the fourth solution further include a surfactant having a percent concentration by volume (vol/vol% or v/v%) of 0.01-2%. Illustratively, the surfactant is a nonionic surfactant, for example, selected from one or more of Tween 20, DD (N,N-dimethyldodecylamine N-oxide), DDM (n-dodecyl-β-D-maltoside), and Triton X-100 (polyethylene glycol octylphenyl ether).

In some embodiments, the surfactant may have a concentration of 0.01%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1%, 1.05%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, or a concentration between any two of these values.

In the embodiments of the present application, S16000 is performed under a variable-temperature condition, S12000 and S14000 are performed under a constant-temperature condition, and a reaction temperature of S16000 is not lower than reaction temperatures of S12000 and S14000.

The inventors of the present application have found through research that the temperature can affect the stability of the secondary structure formed due to high GC content in the template molecule, and a higher temperature is more likely to promote melting of the secondary structure in the template molecule. Based on this finding, the present application promotes the melting of the secondary structure formed due to high GC content in the template molecule by introducing variable-temperature amplification during the constant-temperature amplification, thereby facilitating the binding of the template molecule to the amplification primer and the extension of the amplification primer, improving amplification efficiency, and reducing GC bias.

Specifically, S16000 is performed under a variable-temperature condition, including:
in S16000, performing a first heating on a reaction system of S16000 to raise the temperature of the reaction system of S16000 to a first temperature, and maintaining the first temperature for a first preset duration;
after the first preset duration, performing a second heating on the reaction system of S16000 to raise the temperature of the reaction system of S16000 from the first temperature to a second temperature, and maintaining the second temperature for a second preset duration.

Since the temperature at which the polymerase exhibits optimal activity may be different from the temperature at which the secondary structure melts, the use of a single temperature to complete amplification may not give consideration to both the activity of the polymerase and the melting effect of the secondary structure. Therefore, the present application creatively proposes adopting the above gradient heating for the reaction system of S16000, which can balance the activity of the polymerase in the reaction system and the promoting effect on the melting of the secondary structure formed in the template molecule. For example, supposing that the polymerase exhibits the optimal activity at the first temperature, the temperature, however, does not promote the melting of secondary structures. Therefore, it is conceivable to raise the temperature of the reaction system to a second temperature on the basis of the first temperature and maintain the second temperature for a second preset duration after the first preset duration. As such, the polymerase can maintain good activity to continue to exert its functions, while in terms of the secondary structure in the template molecule, the separation of the two strands is promoted, which facilitates the binding of the template molecule to the amplification primer and the extension of the amplification primer, thereby improving amplification efficiency and reducing GC bias. In some embodiments, the first temperature is 60 °C-65 °C, and the first preset duration is 1-10 s; and/or the second temperature is 80 °C-85 °C, and the second preset duration is 1-10 s. Illustratively, the first temperature may be 60 °C, 61 °C, 62 °C, 63 °C, 64 °C, 65 °C, or a temperature between any two of these values. The first preset duration may be 1 s, 2 s, 3 s, 4 s, 5 s, 6 s, 7 s, 8 s, 9 s, 10 s, or a duration between any two of these values. The second temperature may be 80 °C, 81 °C, 82 °C, 83 °C, 84 °C, 85 °C, or a temperature between any two of these values. The second preset duration may be 1 s, 2 s, 3 s, 4 s, 5 s, 6 s, 7 s, 8 s, 9 s, 10 s, or a duration between any two of these values.

In some embodiments, the first heating rate and the second heating rate are 0.1-5.0 °C/s. Illustratively, the first heating rate and the second heating rate may be 0.1 °C/s, 0.2 °C/s, 0.3 °C/s, 0.4 °C/s, 0.5 °C/s, 0.6 °C/s, 0.7 °C/s, 0.8 °C/s, 0.9 °C/s, 1 °C/s, 1.1 °C/s, 1.2 °C/s, 1.3 °C/s, 1.4 °C/s, 1.5 °C/s, 1.6 °C/s, 1.7 °C/s, 1.8 °C/s, 1.9 °C/s, 2 °C/s, 2.1 °C/s, 2.2 °C/s, 2.3 °C/s, 2.4 °C/s, 2.5 °C/s, 2.6 °C/s, 2.7 °C/s, 2.8 °C/s, 2.9 °C/s, 3 °C/s, 3.1 °C/s, 3.2 °C/s, 3.3 °C/s, 3.4 °C/s, 3.5 °C/s, 3.6 °C/s, 3.7 °C/s, 3.8 °C/s, 3.9 °C/s, 4 °C/s, 4.1 °C/s, 4.2 °C/s, 4.3 °C/s, 4.4 °C/s, 4.5 °C/s, 4.6 °C/s, 4.7 °C/s, 4.8 °C/s, 4.9 °C/s, 5 °C/s, or a rate between any two of these values.

In some embodiments, S16000 is performed under a variable-temperature condition, including:
in S16000, performing at least one pulsed heating on the reaction system of S16000 to raise the temperature of the reaction system of S16000 to a third temperature, where the duration of one pulse is a third preset duration.

By adopting pulsed heating, hydrogen bonds between GCs in the secondary structure formed in the template molecule can be more effectively disrupted, so that the secondary structure is more susceptible to melting, which facilitates the binding of the template molecule to the amplification primer and the extension of the amplification primer, thereby improving amplification efficiency and reducing GC bias.

In some embodiments, the third temperature is 60 °C-85 °C, and the third preset duration is 1-10 s. The third temperature may be 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, 75 °C, 76 °C, 77 °C, 78 °C, 79 °C, 80 °C, 81 °C, 82 °C, 83 °C, 84 °C, 85 °C, or a temperature between any two of these values. The third preset duration may be 1 s, 2 s, 3 s, 4 s, 5 s, 6 s, 7 s, 8 s, 9 s, 10 s, or a duration between any two of these values.

In some embodiments, the pulsed heating has a heating rate of 0.1-5.0 °C/s. The pulsed heating may have a heating rate of 0.1 °C/s, 0.2 °C/s, 0.3 °C/s, 0.4 °C/s, 0.5 °C/s, 0.6 °C/s, 0.7 °C/s, 0.8 °C/s, 0.9 °C/s, 1 °C/s, 1.1 °C/s, 1.2 °C/s, 1.3 °C/s, 1.4 °C/s, 1.5 °C/s, 1.6 °C/s, 1.7 °C/s, 1.8 °C/s, 1.9 °C/s, 2 °C/s, 2.1 °C/s, 2.2 °C/s, 2.3 °C/s, 2.4 °C/s, 2.5 °C/s, 2.6 °C/s, 2.7 °C/s, 2.8 °C/s, 2.9 °C/s, 3 °C/s, 3.1 °C/s, 3.2 °C/s, 3.3 °C/s, 3.4 °C/s, 3.5 °C/s, 3.6 °C/s, 3.7 °C/s, 3.8 °C/s, 3.9 °C/s, 4 °C/s, 4.1 °C/s, 4.2 °C/s, 4.3 °C/s, 4.4 °C/s, 4.5 °C/s, 4.6 °C/s, 4.7 °C/s, 4.8 °C/s, 4.9 °C/s, 5 °C/s, or a rate between any two of these values.

In some embodiments, after completion of one pulse and before performing a next pulse on the reaction system of S16000, the temperature of the reaction system of S16000 is restored to the temperature prior to the pulsed heating. As such, hydrogen bonds between GCs in the secondary structure formed in the template molecule can be repeatedly disrupted to completely open the secondary structure in the template molecule, which facilitates the binding of the template molecule to the amplification primer and the extension of the amplification primer, thereby improving amplification efficiency and reducing GC bias.

Finally, the embodiments of the present application also provide a reagent kit, which includes the first solution, the second solution, the third solution, and the fourth solution in any one of the amplification methods described above.

The subject-matter of the present patent application may be characterized by the following items.

Item 1. An amplification method, comprising:
S20, providing a surface of a solid carrier, wherein the surface of the solid carrier comprises a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution or a second solution to obtain an extended strand of the first amplification primer;
S60, replacing the first solution or the second solution with a third solution to remove the target polynucleotide, wherein the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution or the second solution to obtain an extended strand of the second amplification primer;
S120, replacing the first solution or the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution or the second solution and taking the extended strand of the first amplification primer and the extended strand of the second amplification primer as templates;
S180, repeating S120-S160 at least once,
wherein the first solution, the second solution, and the fourth solution all comprise at least one of betaine, Mg²⁺, NH₄⁺, and formamide, wherein the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume less than or equal to 15%.

Item 2. The amplification method according to item 1, wherein
the betaine has a concentration greater than 0 mol/L and less than or equal to 3 mol/L in the first solution.

Item 3. The amplification method according to item 1 or 2, wherein
the betaine has a concentration of 1.5-2.5 mol/L in the second solution.

Item 4. The amplification method according to any one of items 1-3, wherein
the betaine has a concentration of 1.5-2.5 mol/L in the fourth solution.

Item 5. The amplification method according to any one of items 1-4, wherein
the first solution, the second solution, and the fourth solution all comprise DMSO, the DMSO having a percent concentration by volume of 1-5% in each of the first solution, the second solution, and the fourth solution.

Item 6. The amplification method according to any one of items 1-5, wherein
in S40, the first amplification primer is extended by using the first solution, the Mg²⁺ having a concentration of 2-4 mmol/L in the first solution, and the betaine having a concentration of 0.1-2 mol/L in the first solution.

Item 7. The amplification method according to item 6, wherein
in S100 or S160, the fourth solution is replaced with the second solution, the Mg²⁺ having a concentration of 2-4 mmol/L in the second solution, and the betaine having a concentration of 0.1-2 mol/L in the second solution.

Item 8. The amplification method according to item 6, wherein
in S100 or S160, the fourth solution is replaced with the second solution, the Mg²⁺ having a concentration of 1-1.5 mmol/L in the second solution, and the betaine having a concentration of 2-3 mol/L in the second solution.

Item 9. The amplification method according to any one of items 1-5, wherein
in S40, the first amplification primer is extended by using the second solution, the Mg²⁺ having a concentration of 2-4 mmol/L in the second solution, and the betaine having a concentration of 0.1-2 mol/L in the second solution.

Item 10. The amplification method according to item 9, wherein
in S100 or S160, the fourth solution is replaced with the first solution, the Mg²⁺ having a concentration of 2-4 mmol/L in the first solution, and the betaine having a concentration of 0.1-2 mol/L in the first solution.

Item 11. The amplification method according to item 9, wherein
in S100, the fourth solution is replaced with the first solution, the Mg²⁺ having a concentration of 1-1.5 mmol/L in the first solution, and the betaine having a concentration of 2-3 mol/L in the first solution.

Item 12. The amplification method according to any one of items 1-11, wherein
both the first solution and the second solution comprise dNTPs, the dNTPs having a concentration of 20-1600 µmol/L in the first solution and the second solution.

Item 13. The amplification method according to item 6, 7, 9, or 10, wherein
the first solution or the second solution further comprises dNTPs, the dNTPs comprising dATP, dTTP, dCTP, and dGTP, and a sum of contents of the dATP and the dTTP being equal to a sum of contents of the dGTP and the dCTP.

Item 14. The amplification method according to item 8 or 11, wherein
the second solution or the first solution further comprises dNTPs, the dNTPs comprising dATP, dTTP, dCTP, and dGTP, and a sum of contents of the dATP and the dTTP being less than a sum of contents of the dGTP and the dCTP.

Item 15. The amplification method according to any one of items 1-14, wherein
the NH₄⁺ is NH₄⁺ provided by at least one of ammonium sulfate, ammonium chloride, and ammonium acetate.

Item 16. The amplification method according to any one of items 1-15, wherein
the formamide has a percent concentration by volume greater than or equal to 3% and less than or equal to 15%.

Item 17. The amplification method according to any one of items 1-16, wherein the first solution, the second solution, and the fourth solution further comprise Trisbase, the Trisbase having a concentration of 10-500 mmol/L in each of the first solution, the second solution, and the fourth solution.

Item 18. The amplification method according to item 17, wherein
the Trisbase has a concentration of 10-200 mmol/L in each of the first solution, the second solution, and the fourth solution.

Item 19. The amplification method according to any one of items 1-18, wherein
the first solution, the second solution, and the fourth solution further comprise a single-stranded binding protein and/or bovine serum albumin;
the single-stranded binding protein has a concentration of 0.01-1 mg/mL in the first solution, the second solution, and the fourth solution;
the bovine serum albumin has a percent concentration by volume of 0.05-2% in the first solution, the second solution, and the fourth solution.

Item 20. The amplification method according to any one of items 1-19, wherein
the first solution, the second solution, and the fourth solution further comprise a surfactant, the surfactant having a percent concentration by volume of 0.01-2% in each of the first solution, the second solution, and the fourth solution.

Item 21. The amplification method according to item 20, wherein
the surfactant is a nonionic surfactant.

Item 22. The amplification method according to item 21, wherein
the nonionic surfactant is selected from one or more of Tween 20, DD, DDM, and Triton.

Item 23. The amplification method according to any one of items 1-22, wherein
the first solution, the second solution, and the fourth solution further comprise tetramethylammonium chloride or tetraethylammonium chloride, the tetramethylammonium chloride or tetraethylammonium chloride having a concentration of 10-50 mmol/L in each of the first solution, the second solution, and the fourth solution.

Item 24. The amplification method according to any one of items 1-23, wherein
the denaturing reagent is selected from amides.

Item 25. The amplification method according to item 1 or 24, wherein
the denaturing reagent is formamide, the formamide having a percent concentration by volume of 60-100% in the third solution.

Item 26. The amplification method according to any one of items 1-25, wherein
the first amplification primer and the second amplification primer are oligonucleotides.

Item 27. The amplification method according to any one of items 1-26, wherein
S160 is performed under a variable-temperature condition, S120 and S140 are performed under a constant-temperature condition, and a reaction temperature of S160 is not lower than reaction temperatures of S120 and S140.

Item 28. The amplification method according to item 27, wherein
S160 being performed under the variable-temperature condition comprises:
in S160, performing a first heating on a reaction system of S160 to raise the temperature of the reaction system of S160 to a first temperature, and maintaining the first temperature for a first preset duration;
after the first preset duration, performing a second heating on the reaction system of S160 to raise the temperature of the reaction system of S160 from the first temperature to a second temperature, and maintaining the second temperature for a second preset duration.

Item 29. The amplification method according to item 28, wherein
the first temperature is 60 °C-65 °C, and the first preset duration is 1-10 s; and/or,
the second temperature is 80 °C-85 °C, and the second preset duration is 1-10 s.

Item 30. The amplification method according to item 28 or 29, wherein
the first heating and the second heating have a heating rate of 0.1-5.0 °C/s.

Item 31. The amplification method according to any one of items 27-30, wherein
S160 being performed under the variable-temperature condition comprises:
in S160, performing at least one pulsed heating on the reaction system of S160 to raise the temperature of the reaction system of S160 to a third temperature,
wherein a duration of one pulse is a third preset duration.

Item 32. The amplification method according to item 31, wherein
the third temperature is 60 °C-85 °C, and the third preset duration is 1-10 s.

Item 33. The amplification method according to item 31 or 32, wherein the pulsed heating has a heating rate of 0.1-5.0 °C/s.

Item 34. The amplification method according to any one of items 31-33, wherein
after completion of one pulse and before performing a next pulse on the reaction system of S160, the temperature of the reaction system of S160 is restored to the temperature prior to the pulsed heating. Item 35. An amplification method, comprising:
S200, providing a surface of a solid carrier, wherein the surface of the solid carrier comprises a first amplification primer, a second amplification primer, and a target polynucleotide that are immobilized thereon, at least a portion of the target polynucleotide complementarily hybridizing with the first amplification primer;
S400, extending the first amplification primer by taking the target polynucleotide as a template and using the first solution or the second solution to obtain an extended strand of the first amplification primer;
S600, replacing the first solution or the second solution with a third solution to remove the target polynucleotide, wherein the third solution contains a denaturing reagent;
S800, replacing the third solution with a fourth solution to allow the extended strand of the first amplification primer to bind to the second amplification primer;
S1000, extending the second amplification primer by taking the extended strand of the first amplification primer as a template and replacing the fourth solution with the first solution or the second solution to obtain an extended strand of the second amplification primer;
S1200, replacing the first solution or the second solution with the third solution;
S1400, replacing the third solution with the fourth solution to allow the extended strand of the first amplification primer and the extended strand of the second amplification primer to bind to the second amplification primer and the first amplification primer, respectively;
S1600, extending the first amplification primer and the second amplification primer by replacing the fourth solution with the first solution or the second solution and taking the extended strand of the first amplification primer and the extended strand of the second amplification primer as templates;
S1800, repeating S1200-S1600 at least once,
wherein S1600 is performed under a variable-temperature condition, S1200 and S1400 are performed under a constant-temperature condition, and a reaction temperature of S1600 is not lower than reaction temperatures of S1200 and S1400.

Item 36. The amplification method according to item 35, wherein
S1600 being performed under the variable-temperature condition comprises:
in S1600, performing a first heating on a reaction system of S1600 to raise the temperature of the reaction system of S1600 to a first temperature, and maintaining the first temperature for a first preset duration;
after the first preset duration, performing a second heating on the reaction system of S1600 to raise the temperature of the reaction system of S1600 from the first temperature to a second temperature, and maintaining the second temperature for a second preset duration.

Item 37. The amplification method according to claim 36, wherein
the first temperature is 60 °C-65 °C, and the first preset duration is 1-10 s;
the second temperature is 80 °C-85 °C, and the second preset duration is 1-10 s.

Item 38. The amplification method according to claim 36 or 37, wherein
the first heating and the second heating have a heating rate of 0.1-5.0 °C/s.

Item 39. The amplification method according to any one of items 35-38, wherein
S1600 being performed under the variable-temperature condition comprises:
in S1600, performing at least one pulsed heating on the reaction system of S1600 to raise the temperature of the reaction system of S1600 to a third temperature,
wherein a duration of one pulse is a third preset duration.

Item 40. The amplification method according to item 39, wherein
the third temperature is 60 °C-85 °C, and the third preset duration is 1-10 s.

Item 41. The amplification method according to item 39 or 40, wherein the pulsed heating has a heating rate of 0.1-5.0 °C/s.

Item 42. The amplification method according to any one of items 39-41, wherein
after completion of one pulse and before performing a next pulse on the reaction system of S1600, the temperature of the reaction system of S1600 is restored to the temperature prior to the pulsed heating. Item 43. The amplification method according to any one of items 35-42, wherein
the first solution, the second solution, and the fourth solution all comprise at least one of betaine, Mg²⁺, NH₄⁺, and formamide, wherein the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume less than or equal to 15%.

Item 44. The amplification method according to item 43, wherein
the betaine has a concentration greater than 0 mol/L and less than or equal to 3 mol/L in the first solution.

Item 45. The amplification method according to item 42 or 43, wherein
the betaine has a concentration of 1.5-2.5 mol/L in the second solution.

Item 46. The amplification method according to any one of items 43-45, wherein the betaine has a concentration of 1.5-2.5 mol/L in the fourth solution.

Item 47. The amplification method according to any one of items 43-46, wherein
the first solution, the second solution, and the fourth solution all comprise DMSO, the DMSO having a percent concentration by volume of 1-5% in each of the first solution, the second solution, and the fourth solution.

Item 48. The amplification method according to any one of items 43-47, wherein
in S400, the first amplification primer is extended by using the first solution, the Mg²⁺ having a concentration of 2-4 mmol/L in the first solution, and the betaine having a concentration of 0.1-2 mol/L in the first solution.

Item 49. The amplification method according to item 48, wherein
in S1000 or S1600, the fourth solution is replaced with the second solution, the Mg²⁺ having a concentration of 2-4 mmol/L in the second solution, and the betaine having a concentration of 0.1-2 mol/L in the second solution.

Item 50. The amplification method according to item 48, wherein
in S1000 or S1600, the fourth solution is replaced with the second solution, the Mg²⁺ having a concentration of 1-1.5 mmol/L in the second solution, and the betaine having a concentration of 2-3 mol/L in the second solution.

Item 51. The amplification method according to any one of items 43-47, wherein
in S400, the first amplification primer is extended by using the second solution, the Mg²⁺ having a concentration of 2-4 mmol/L in the second solution, and the betaine having a concentration of 0.1-2 mol/L in the second solution.

Item 52. The amplification method according to item 51, wherein
in S1000 or S1600, the second amplification primer is extended by replacing the fourth solution with the first solution, the Mg²⁺ having a concentration of 2-4 mmol/L in the first solution, and the betaine having a concentration of 0.1-2 mol/L in the first solution.

Item 53. The amplification method according to item 51, wherein
in S1000 or S1600, the second amplification primer is extended by replacing the fourth solution with the first solution, the Mg²⁺ having a concentration of 1-1.5 mmol/L in the first solution, and the betaine having a concentration of 2-3 mol/L in the first solution.

Item 54. The amplification method according to any one of items 35-53, wherein
both the first solution and the second solution comprise dNTPs, the dNTPs having a concentration of 20-1600 µmol/L in the first solution and the second solution.

Item 55. The amplification method according to item 48, 49, 51, or 52, wherein
the first solution or the second solution further comprises dNTPs, the dNTPs comprising dATP, dTTP, dCTP, and dGTP, and a sum of contents of the dATP and the dTTP being equal to a sum of contents of the dGTP and the dCTP.

Item 56. The amplification method according to item 50 or 53, wherein
the second solution or the first solution further comprises dNTPs, the dNTPs comprising dATP, dTTP, dCTP, and dGTP, and a sum of contents of the dATP and the dTTP being less than a sum of contents of the dGTP and the dCTP.

Item 57. The amplification method according to any one of items 35-56, wherein
the NH₄⁺ is NH₄⁺ provided by at least one of ammonium sulfate, ammonium chloride, and ammonium acetate.

Item 58. The amplification method according to any one of items 35-57, wherein
the first solution, the second solution, and the fourth solution further comprise Trisbase, the Trisbase having a concentration of 10-500 mmol/L in each of the first solution, the second solution, and the fourth solution.

Item 59. The amplification method according to item 58, wherein
the Trisbase has a concentration of 10-200 mmol/L in each of the first solution, the second solution, and the fourth solution.

Item 60. The amplification method according to any one of items 35-59, wherein
the first solution, the second solution, and the fourth solution further comprise a single-stranded binding protein and/or bovine serum albumin;
the single-stranded binding protein has a concentration of 0.01-1 mg/mL in the first solution, the second solution, and the fourth solution;
the bovine serum albumin has a percent concentration by volume of 0.05-2% in the first solution, the second solution, and the fourth solution.

Item 61. The amplification method according to any one of items 35-60, wherein
the first solution, the second solution, and the fourth solution further comprise a surfactant, the surfactant having a percent concentration by volume of 0.01-2% in each of the first solution, the second solution, and the fourth solution.

Item 62. The amplification method according to item 61, wherein
the surfactant is a nonionic surfactant.

Item 63. The amplification method according to item 62, wherein
the nonionic surfactant is selected from one or more of Tween 20, DD, DDM, and Triton.

Item 64. The amplification method according to any one of items 35-63, wherein
the first solution, the second solution, and the fourth solution further comprise tetramethylammonium chloride or tetraethylammonium chloride, the tetramethylammonium chloride or tetraethylammonium chloride having a concentration of 10-50 mmol/L in each of the first solution, the second solution, and the fourth solution.

Item 65. The amplification method according to any one of items 35-64, wherein
the denaturing reagent is selected from amides.

Item 66. The amplification method according to item 35 or 65, wherein
the denaturing reagent is formamide, the formamide having a percent concentration by volume of 60-100% in the third solution.

Item 67. The amplification method according to any one of items 35-66, wherein
the first amplification primer and the second amplification primer are oligonucleotides.

Item 68. An amplification method, comprising:
S2000, providing a surface of a solid carrier, wherein the surface of the solid carrier comprises a first amplification primer, a second amplification primer, and a target polynucleotide that are immobilized thereon, at least a portion of the target polynucleotide complementarily hybridizing with the first amplification primer;
S4000, extending the first amplification primer by taking the target polynucleotide as a template and using a first solution or a second solution to obtain an extended strand of the first amplification primer;
S6000, replacing the first solution or the second solution with a third solution to remove the target polynucleotide, wherein the third solution contains a denaturing reagent;
S8000, replacing the third solution with a fourth solution to allow the extended strand of the first amplification primer to bind to the second amplification primer;
S10000, extending the second amplification primer by taking the extended strand of the first amplification primer as a template and replacing the fourth solution with the first solution or the second solution to obtain an extended strand of the second amplification primer;
S12000, replacing the first solution or the second solution with the third solution;
S14000, replacing the third solution with the fourth solution to allow the extended strand of the first amplification primer and the extended strand of the second amplification primer to bind to the second amplification primer and the first amplification primer, respectively;
S16000, extending the first amplification primer and the second amplification primer by replacing the fourth solution with the first solution or the second solution and taking the extended strand of the first amplification primer and the extended strand of the second amplification primer as templates;
S18000, repeating S12000-S16000 at least once,
wherein in S10000 and S16000, the first solution or the second solution comprises dATP, dTTP, dCTP, and dGTP, and a sum of contents of the dATP and the dTTP is less than a sum of contents of the dGTP and the dCTP.

Item 69. The amplification method according to item 68, wherein
the first solution, the second solution, and the fourth solution all comprise at least one of betaine, Mg²⁺, NH₄⁺, and formamide, wherein the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume less than or equal to 15%.

Item 70. The amplification method according to item 69, wherein
in S4000, the first amplification primer is extended by using the first solution, and in S10000 and S16000, the fourth solution is replaced with the first solution or the second solution, wherein in the first solution, the Mg²⁺ has a concentration of 2-4 mmol/L, and the betaine has a concentration of 0.1-2 mol/L, and in the second solution, the Mg²⁺ has a concentration of 2-4 mmol/L, and the betaine has a concentration of 0.1-2 mol/L.

Item 71. The amplification method according to item 69, wherein
in S4000, the first amplification primer is extended by using the first solution, and in S10000 and S16000, the fourth solution is replaced with the first solution or the second solution, wherein in the first solution, the Mg²⁺ has a concentration of 2-4 mmol/L, and the betaine has a concentration of 0.1-2 mol/L, and in the second solution, the Mg²⁺ has a concentration of 1-1.55 mmol/L, and the betaine has a concentration of 2-3 mol/L.

Item 72. The amplification method according to item 69, wherein
in S4000, the first amplification primer is extended by using the second solution; and in S10000 and S16000, the fourth solution is replaced with the first solution or the second solution, wherein in the first solution, the Mg²⁺ has a concentration of 2-4 mmol/L, and the betaine has a concentration of 0.1-2 mol/L, and in the second solution, the Mg²⁺ has a concentration of 2-4 mmol/L, and the betaine has a concentration of 0.1-2 mol/L.

Item 73. The amplification method according to item 69, wherein
in S4000, the first amplification primer is extended by using the second solution; and in S10000 and S16000, the second amplification primer is extended by replacing the fourth solution with the first solution or the second solution, wherein in the first solution, the Mg²⁺ has a concentration of 1-1.5 mmol/L, and the betaine has a concentration of 2-3 mol/L, and in the second solution, the Mg²⁺ has a concentration of 2-4 mmol/L, and the betaine has a concentration of 0.1-2 mol/L.

Item 74. The amplification method according to any one of items 68-73, wherein
in S4000, the first solution or the second solution comprises dATP, dTTP, dCTP, and dGTP, and a sum of contents of the dATP and the dTTP is equal to a sum of contents of the dGTP and the dCTP.

Item 75. The amplification method according to any one of items 69-74, wherein
the betaine has a concentration of 1.5-2.5 mol/L in the fourth solution.

Item 76. The amplification method according to any one of items 69-75, wherein
the NH₄⁺ is NH₄⁺ provided by at least one of ammonium sulfate, ammonium chloride, and ammonium acetate.

Item 77. The amplification method according to any one of items 68-76, wherein
the first solution, the second solution, and the fourth solution further comprise Trisbase, the Trisbase having a concentration of 10-500 mmol/L in each of the first solution, the second solution, and the fourth solution.

Item 78. The amplification method according to item 77, wherein
the Trisbase has a concentration of 10-200 mmol/L in each of the first solution, the second solution, and the fourth solution.

Item 79. The amplification method according to any one of items 68-78, wherein
the first solution, the second solution, and the fourth solution further comprise a single-stranded binding protein and/or bovine serum albumin;
the single-stranded binding protein has a concentration of 0.01-1 mg/mL in the first solution, the second solution, and the fourth solution;
the bovine serum albumin has a percent concentration by volume of 0.05-2% in the first solution, the second solution, and the fourth solution.

Item 80. The amplification method according to any one of items 68-79, wherein
the first solution, the second solution, and the fourth solution further comprise a surfactant, the surfactant having a percent concentration by volume of 0.01-2% in each of the first solution, the second solution, and the fourth solution.

Item 81. The amplification method according to item 80, wherein
the surfactant is a nonionic surfactant.

Item 82. The amplification method according to item 81, wherein
the nonionic surfactant is selected from one or more of Tween 20, DD, DDM, and Triton.

Item 83. The amplification method according to any one of items 68-82, wherein
the first solution, the second solution, and the fourth solution further comprise tetramethylammonium chloride or tetraethylammonium chloride, the tetramethylammonium chloride or tetraethylammonium chloride having a concentration of 10-50 mmol/L in each of the first solution, the second solution, and the fourth solution.

Item 84. The amplification method according to any one of items 68-83, wherein
the denaturing reagent is selected from amides.

Item 85. The amplification method according to item 68 or 84, wherein
the denaturing reagent is formamide, the formamide having a percent concentration by volume of 60-100% in the third solution.

Item 86. The amplification method according to any one of items 68-85, wherein
the first amplification primer and the second amplification primer are oligonucleotides.

Item 87. The amplification method according to any one of items 68-86, wherein
S16000 is performed under a variable-temperature condition, S12000 and S14000 are performed under a constant-temperature condition, and a reaction temperature of S16000 is not lower than reaction temperatures of S12000 and S14000.

Item 88. The amplification method according to item 87, wherein
S16000 being performed under the variable-temperature condition comprises:
in S16000, performing a first heating on a reaction system of S16000 to raise the temperature of the reaction system of S16000 to a first temperature, and maintaining the first temperature for a first preset duration;
after the first preset duration, performing a second heating on the reaction system of S16000 to raise the temperature of the reaction system of S16000 from the first temperature to a second temperature, and maintaining the second temperature for a second preset duration.

Item 89. The amplification method according to item 88, wherein
the first temperature is 60 °C-65 °C, and the first preset duration is 1-10 s;
the second temperature is 80 °C-85 °C, and the second preset duration is 1-10 s.

Item 90. The amplification method according to item 88 or 89, wherein
the first heating and the second heating have a heating rate of 0.1-5.0 °C/s.

Item 91. The amplification method according to any one of items 87-90, wherein
S16000 being performed under the variable-temperature condition comprises:
in S16000, performing at least one pulsed heating on the reaction system of S16000 to raise the temperature of the reaction system of S16000 to a third temperature,
wherein a duration of one pulse is a third preset duration.

Item 92. The amplification method according to item 91, wherein
the third temperature is 60 °C-85 °C, and the third preset duration is 1-10 s.

Item 93. The amplification method according to item 91 or 92, wherein
the pulsed heating has a heating rate of 0.1-5.0 °C/s.

Item 94. The amplification method according to any one of items 91-93, wherein
after completion of one pulse and before performing a next pulse on the reaction system of S16000, the temperature of the reaction system of S16000 is restored to the temperature prior to the pulsed heating. Item 95. A reagent kit, comprising the first solution, the second solution, the third solution, and the fourth solution of the amplification method according to any one of items 1-94.

The present application will be illustrated with reference to the following specific examples. The experimental instruments and their manufacturers involved in the examples of the present application are shown in Table 1:

**Table 1**

| | | |
|---|---|---|
| Experimental instrument | Model | Manufacturer |
| FASTASeq300 sequencer | FASTASeq300 | Shenzhen GeneMind Biosciences Co., Ltd. |

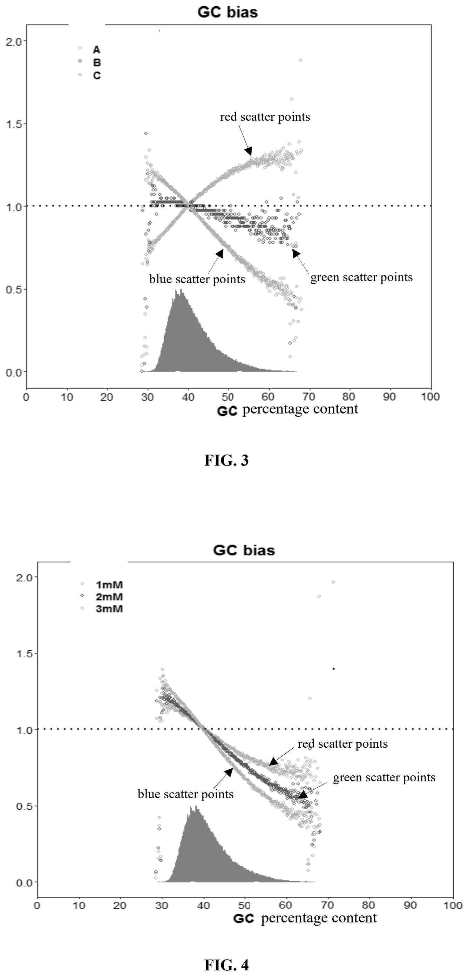

As shown in FIG. 3, generally, the GC bias in the sequencing results can be visually displayed through a GC bias scatter plot. The abscissa of the diagram represents the GC percentage content of the target polynucleotide, and the ordinate represents the ratio of the sequencing abundance to the true abundance of the target polynucleotide. Specifically, if it is hypothesized that the sequencing results have no bias, then the ratios of sequencing abundance to true abundance for target polynucleotides with different GC contents will all be around 1, and the corresponding scatter plot should exhibit a nearly horizontal distribution, as shown by the green scatter points; if it is hypothesized that the sequencing results are biased toward high GC, then the ratios of sequencing abundance to true abundance for target polynucleotides with a high GC content will be greater than 1, and the corresponding scatter plot should exhibit an upward distribution, as shown by the red scatter points; if it is hypothesized that the sequencing results are biased toward low GC, then the ratios of sequencing abundance to true abundance for target polynucleotides with a low GC content will be less than 1, and the corresponding scatter plot should exhibit a downward distribution, as shown by the blue scatter points; the more horizontal the trend of the scatter plot, the lower the GC bias, and the more inclined the trend, the higher the GC bias.

### Example 1

In Example 1 of the present application, the FASTASeq300 sequencer was used; amplification was performed in a constant-temperature amplification mode (at an amplification temperature of 55 °C) according to the following amplification method, followed by sequencing:
S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution to obtain an extended strand of the second amplification primer;
S120, replacing the first solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution and taking the extended strand of the first amplification primer and the extended strand of the second amplification primer as templates;
S180, repeating S120-S160 30 times.

The first solution included DMSO having a percent concentration by volume (vol/vol% or v/v%) of 5%, 2.5 mol/L betaine, 2.5 mmol/L Mg²⁺, formamide having a percent concentration by volume (vol/vol% or v/v%) of 3%, 200 mmol/L Trisbase, 50 mmol/L ammonium chloride, 1.5 mg/mL BST polymerase, and 200 mmol/L each of dATP, dTTP, dCTP, and dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 2.5 mol/L betaine, 2.5 mmol/L Mg²⁺, formamide having a percent concentration by volume (vol/vol% or v/v%) of 3%, 200 mmol/L Trisbase, and 50 mmol/L ammonium chloride.

### Comparative Example 1-1

Comparative Example 1-1 investigated the effect of different Mg²⁺ concentrations on GC bias. In Comparative Example 1-1, only the Mg²⁺ concentration in the first solution and the fourth solution was adjusted on the basis of Example 1. In Comparative Example 1-1, the Mg²⁺ concentration in both the first solution and the fourth solution was 1 mmol/L, 2 mmol/L, or 3 mmol/L. The results are shown in FIG. 4: the red, green, and blue scatter points represent the GC bias scatter plots when the Mg²⁺ concentration in the first solution and the fourth solution was 1 mmol/L, 2 mmol/L, and 3 mmol/L, respectively. As can be seen from FIG. 4, as the GC content on the target polynucleotide gradually increased, the sequencing abundance decreased, indicating the presence of GC bias in the sequencing results; with the addition of Mg²⁺ during the amplification, as the Mg²⁺ concentration decreased, the GC bias scatter plot tended to become more horizontal, and the GC bias decreased.

### Comparative Example 1-2

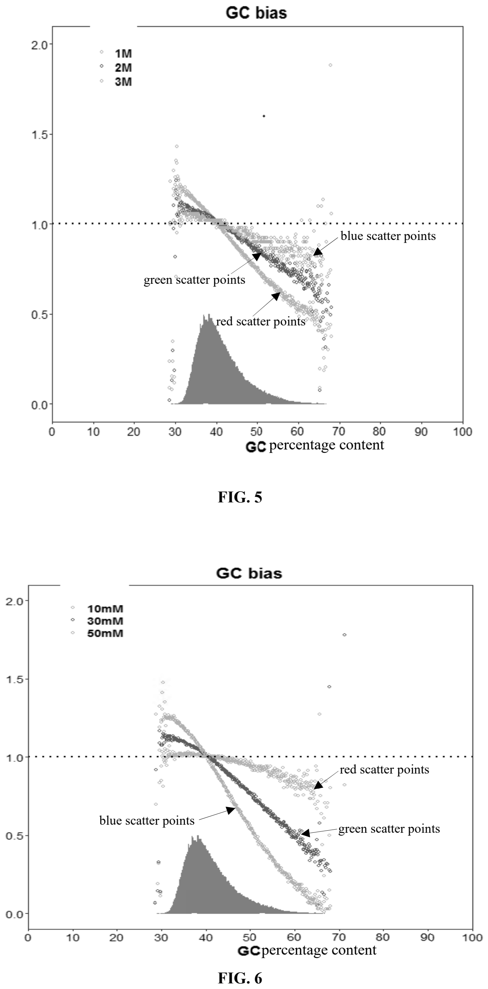

Comparative Example 1-2 investigated the effect of different betaine concentrations on GC bias. In Comparative Example 1-2, only the betaine concentration in the first solution and the fourth solution was adjusted on the basis of Example 1. In Comparative Example 1-2, the betaine concentration in both the first solution and the fourth solution was 1 mol/L, 2 mol/L, or 3 mol/L. The results are shown in FIG. 5: the red, green, and blue scatter points represent the GC bias scatter plots when the betaine concentration in the first solution and the fourth solution was 1 mol/L, 2 mol/L, and 3 mol/L, respectively. As can be seen from FIG. 5, as the GC content on the target polynucleotide gradually increased, the sequencing abundance decreased, indicating the presence of GC bias in the sequencing results; with the addition of betaine during the amplification, as the betaine concentration increased, the GC bias scatter plot tended to become more horizontal, and the GC bias decreased.

### Comparative Example 1-3

Comparative Example 1-3 investigated the effect of different NH₄⁺ concentrations on GC bias. In Comparative Example 1-3, only the NH₄⁺ concentration in the first solution and the fourth solution was adjusted on the basis of Example 1. In Comparative Example 1-3, the NH₄⁺ concentration in both the first solution and the fourth solution was 10 mmol/L, 30 mmol/L, or 50 mmol/L. The results are shown in FIG. 6: the red, green, and blue scatter points represent the GC bias scatter plots when the NH₄⁺ concentration in the first solution and the fourth solution was 10 mmol/L, 30 mmol/L, and 50 mmol/L, respectively. As can be seen from FIG. 6, as the GC content on the target polynucleotide gradually increased, the sequencing abundance decreased, indicating the presence of GC bias in the sequencing results; with the addition of NH₄⁺ during the amplification, as the NH₄⁺ concentration increased, the GC bias scatter plot tended to become more horizontal, and the GC bias decreased.

### Example 2

In Example 2 of the present application, the FASTASeq300 sequencer was used; amplification was performed in a constant-temperature amplification mode (at an amplification temperature of 55 °C) according to the following amplification method, followed by sequencing:
S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution to obtain an extended strand of the second amplification primer;
S120, replacing the first solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution and taking the extended strand of the first amplification primer and the extended strand of the second amplification primer as templates;
S180, repeating S120-S160 30 times.

The first solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 100 mmol/L Trisbase, 100 mmol/L ammonium chloride, 1.5 mg/mL BST polymerase, and 200 mmol/L each of dATP, dTTP, dCTP, and dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 100 mmol/L Trisbase, and 100 mmol/L ammonium chloride.

### Comparative Example 2-1

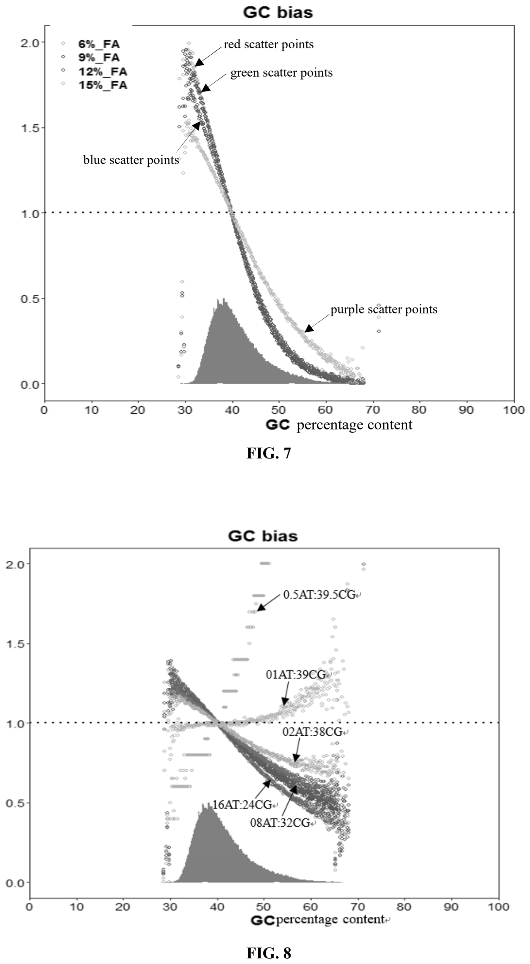

Comparative Example 2-1 investigated the effect of different formamide concentrations on GC bias. In Comparative Example 2-1, only the formamide concentration in the first solution and the fourth solution was adjusted on the basis of Example 2. In Comparative Example 2-1, the formamide concentration in both the first solution and the fourth solution was 6%, 9%, 12%, or 15%. The results are shown in FIG. 7: the red, green, blue and purple scatter points represent the GC bias scatter plots when the formamide concentration in the first solution and the fourth solution was 6%, 9%, 12%, and 15%, respectively. As can be seen from FIG. 7, as the GC content on the target polynucleotide gradually increased, the sequencing abundance decreased, indicating the presence of GC bias in the sequencing results; with the addition of formamide during the amplification, as the formamide concentration increased, the GC bias scatter plot tended to become more horizontal, and the GC bias decreased.

### Example 3

In Example 3 of the present application, the FASTASeq300 sequencer was used; amplification was performed in a constant-temperature amplification mode (at an amplification temperature of 50 °C) according to the following amplification method, followed by sequencing:
S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the second solution to obtain an extended strand of the second amplification primer;
S120, replacing the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the second solution and taking the extended strand of the first amplification primer and the extended strand of the second amplification primer as templates;
S180, repeating S120-S160 30 times.

The first solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 50 mmol/L Trisbase, 30 mmol/L ammonium chloride, 1.5 mg/mL BST polymerase, and 200 mmol/L each of dATP, dTTP, dCTP, and dGTP;
the second solution included DMSO having a percent concentration by volume (vol/vol% or v/v%) of 5%, 2 mol/L betaine, 1 mmol/L Mg²⁺, formamide having a percent concentration by volume (vol/vol% or v/v%) of 3%, 10 mmol/L Trisbase, 30 mmol/L ammonium chloride, 1.5 mg/mL BST polymerase, and 200 mmol/L each of dATP, dTTP, dCTP, and dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 2 mol/L betaine, 1 mmol/L Mg²⁺, formamide having a percent concentration by volume (vol/vol% or v/v%) of 3%, 10 mmol/L Trisbase, and 30 mmol/L ammonium chloride.

### Comparative Example 3-1

The comparative example investigated the effect of different AT contents and GC contents on GC bias. In Comparative Example 3-1, only the concentrations of dATP, dTTP, dCTP, and dGTP in the first solution and the second solution were adjusted on the basis of Example 3. In Comparative Example 3-1, the concentrations of dATP, dTTP, dCTP, and dGTP in the first solution and the second solution are shown in Table 2 below (the content in the rightmost remark in Table 2 can be simply understood as the ratio of the total concentration of dATP and dTTP to the total concentration of dCTP and dGTP; for example, 0.5AT:39.5CG indicates that the ratio of the total concentration of dATP and dTTP to the total concentration of dCTP and dGTP is 0.5:39.5, and so on). The results are shown in FIG. 8. As can be seen from FIG. 8, as the GC content on the target polynucleotide gradually increased, the sequencing abundance decreased, indicating the presence of GC bias in the sequencing results; however, under the condition that the total nucleotide concentration was kept unchanged during the amplification, as the concentrations of dCTP and dGTP increased, the GC bias scatter plot gradually tended to become horizontal and ultimately tilted upward, and the GC bias first decreased and then increased, which may be related to a gradual shift during the amplification from amplification biased towards low-GC templates to amplification biased towards high-GC templates.

**Table 2**

| Serial number | Concentration: µmol/L | | | | Remark |
|---|---|---|---|---|---|
| | dATP | dTTP | dCTP | dGTP | |
| 1 | 5 | 5 | 395 | 395 | 0.5AT:39.5CG |
| 2 | 10 | 10 | 390 | 390 | 01AT:39CG |
| 3 | 20 | 20 | 380 | 380 | 02AT:38CG |
| 4 | 30 | 30 | 370 | 370 | 03AT:37CG |
| 5 | 40 | 40 | 360 | 360 | 04AT:36CG |
| 6 | 80 | 80 | 320 | 320 | 08AT:32CG |
| 7 | 120 | 120 | 280 | 280 | 12AT:28CG |
| 8 | 160 | 160 | 240 | 240 | 16AT:24CG |
| 9 | 200 | 200 | 200 | 200 | 20AT:20CG |

### Example 4

In Example 4 of the present application, the FASTASeq300 sequencer was used; amplification was performed in a constant-temperature amplification mode (at an amplification temperature of 50 °C) according to the amplification methods in the following 4 schemes (scheme A, scheme B, scheme C, scheme D), respectively, followed by sequencing:

### Scheme A:

S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the second solution to obtain an extended strand of the second amplification primer;
S120, replacing the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the second solution and taking the extended strand of the first amplification primer and the extended strand of the second amplification primer as templates;
S180, repeating S120-S160 27 times.

The first solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 50 mmol/L Trisbase, 30 mmol/L ammonium chloride, 1.5 mg/mL BST polymerase, and 200 mmol/L each of dATP, dTTP, dCTP, and dGTP;
the second solution included DMSO having a percent concentration by volume (vol/vol% or v/v%) of 5%, 2 mol/L betaine, 1 mmol/L Mg²⁺, formamide having a percent concentration by volume (vol/vol% or v/v%) of 3%, 10 mmol/L Trisbase, 30 mmol/L ammonium chloride, 1.5 mg/mL BST polymerase, 10 µmol/L dATP, 10 µmol/L dTTP, 390 µmol/L dCTP, and 390 µmol/L dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 2 mol/L betaine, 1 mmol/L Mg²⁺, formamide having a percent concentration by volume (vol/vol% or v/v%) of 3%, 10 mmol/L Trisbase, and 30 mmol/L ammonium chloride.

### Scheme B:

S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution to obtain an extended strand of the second amplification primer;
S120, replacing the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution and taking the extended strand of the first amplification primer and the extended strand of the second amplification primer as templates;
S180, repeating S120-S160 27 times.

The first solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 50 mmol/L Trisbase, 30 mmol/L ammonium chloride, 1.5 mg/mL BST polymerase, and 200 mmol/L each of dATP, dTTP, dCTP, and dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 2 mol/L betaine, 1 mmol/L Mg²⁺, formamide having a percent concentration by volume (vol/vol% or v/v%) of 3%, 10 mmol/L Trisbase, and 30 mmol/L ammonium chloride.

### Scheme C

The difference between the amplification method of scheme C and those of scheme A and scheme B is as follows:
Scheme C was first performed according to the amplification method of scheme A with S120-S160 repeated 17 times, and then S120-S160 in scheme B were repeated 10 times.

### Scheme D

The difference between the amplification method of scheme D and those of scheme A and scheme B is as follows:
Scheme D was first performed according to the amplification method of scheme A with S120-S160 repeated 22 times. and then S120-S160 in scheme B were repeated 5 times.

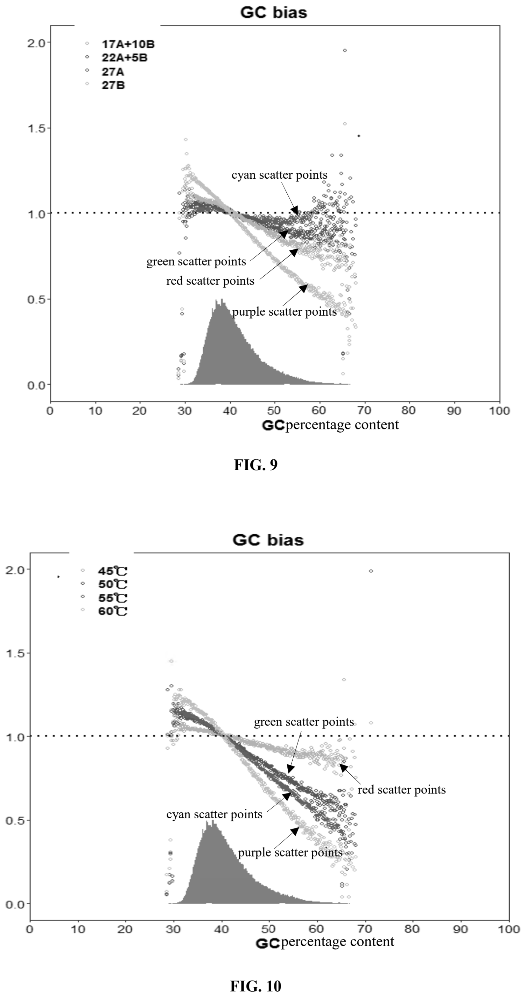

The results are shown in FIG. 9, where:
Cyan represents the GC bias scatter plot for amplification performed using scheme A;
Purple represents the GC bias scatter plot for amplification performed using scheme B;
Red represents the GC bias scatter plot for amplification performed using scheme C;
Green represents the GC bias scatter plot for amplification performed using scheme D.

As can be seen from the figure, as the GC content on the target polynucleotide gradually increased, the sequencing abundance decreased, indicating the presence of GC bias in the sequencing results; however, as the number of cycles using scheme A during the amplification increased, the scatter plot tended to become more horizontal, and the GC bias gradually decreased. This indicates that introducing an imbalanced base ratio during the cyclic amplification, i.e., the sum of the contents of dATP and dTTP being less than the sum of the contents of dGTP and dCTP, can reduce GC bias.

### Example 5

In Example 5 of the present application, the FASTASeq300 sequencer was used; amplification was performed in a constant-temperature amplification mode (at an amplification temperature of 50 °C) according to the following amplification method, followed by sequencing:
S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution to obtain an extended strand of the second amplification primer;
S120, replacing the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution and taking the extended strand of the first amplification primer and the extended strand of the second amplification primer as templates;
S180, repeating S120-S160 30 times.

The first solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 100 mmol/L Trisbase, 100 mmol/L ammonium chloride, 1.5 mg/mL BST polymerase, and 200 mmol/L each of dATP, dTTP, dCTP, and dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 100 mmol/L Trisbase, and 100 mmol/L ammonium chloride.

### Comparative Example 5-1

Comparative Example 5-1 investigated the effect of the temperature for constant-temperature amplification on GC bias. In Comparative Example 5-1, only the temperature for constant-temperature amplification was adjusted on the basis of Example 5. The results are shown in FIG. 10, where: purple, cyan, green, and red represent the GC bias scatter plots for amplification performed at temperatures of 60 °C, 55 °C, 50 °C, and 45 °C, respectively. As can be seen from the figure, as the GC content on the target polynucleotide gradually increased, the sequencing abundance decreased, indicating the presence of GC bias in the sequencing results; however, as the amplification temperature gradually decreased, the scatter plot tended to become more horizontal, and the GC bias gradually decreased.

### Example 6

In Example 6 of the present application, the FASTASeq300 sequencer was used; amplification was performed in a constant-temperature amplification mode (at an amplification temperature of 50 °C) according to the following amplification method, and pulsed heating was introduced during the amplification, followed by sequencing:
S20, providing a surface of a solid carrier, which includes a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution to obtain an extended strand of the first amplification primer;
S60, replacing the first solution with a third solution to remove the target polynucleotide, where the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution to obtain an extended strand of the second amplification primer;
S120, replacing the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution and taking the extended strand of the first amplification primer and the extended strand of the second amplification primer as templates;
S180, repeating S120-S160 30 times.

The first solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 100 mmol/L Trisbase, 100 mmol/L ammonium chloride, 1.5 mg/mL BST polymerase, 4 U/mL rTaq enzyme, and 200 mmol/L each of dATP, dTTP, dCTP, and dGTP;
the third solution included formamide having a percent concentration by volume (vol/vol% or v/v%) of 90% and Tween 20 having a percent concentration by volume (vol/vol% or v/v%) of 0.05%;
the fourth solution included DMSO having a percent concentration by volume (vol/vol% or v/v%) of 5%, 1 mol/L betaine, 2.5 mmol/L Mg²⁺, formamide having a percent concentration by volume (vol/vol% or v/v%) of 3%, 100 mmol/L Trisbase, and 100 mmol/L ammonium chloride.

### Comparative Example 6-1

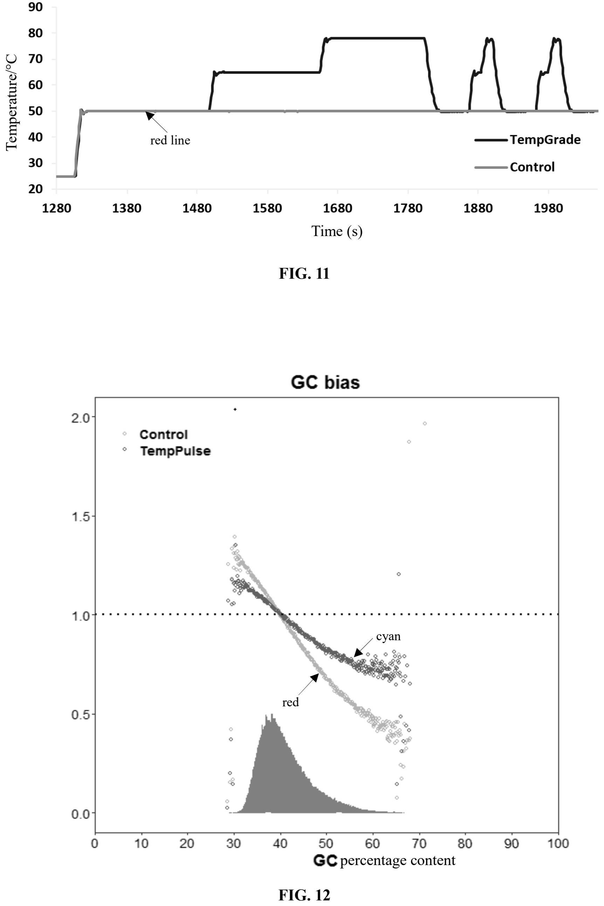

Comparative Example 6-1 investigated the effect of the introduction of pulsed heating during a constant-temperature process on GC bias. The curves for the comparison between the constant-temperature amplification and the pulsed heating amplification are shown in FIG. 11. The constant-temperature amplification was set at 50 °C (red line), and one cycle of pulsed heating was set to 2 pulses with pulse temperatures of 65 °C and 80 °C, respectively. The GC bias scatter plots are shown in FIG. 12: red and cyan represent constant-temperature amplification and constant-temperature amplification with introduced pulsed heating, respectively. As can be seen from the figure, as the GC content on the target polynucleotide gradually increased, the sequencing abundance decreased, indicating the presence of GC bias in the sequencing results. However, after introducing pulsed heating during the constant-temperature amplification, the scatter plot tended to become more horizontal, and the GC bias gradually decreased.

In the description of the present application, the description of the terms "one embodiment", "some embodiments", "schematic embodiments", "examples", "certain examples", "specific examples", or the like, means that the particular features, structures, materials, or characteristics described with reference to the embodiment or example are included in at least one embodiment or example of the present application. In the present application, the schematic description of the terms described above does not necessarily refer to the same embodiment or example. Moreover, the particular features, structures, materials, or characteristics described may be combined in any one or more embodiments or examples in any appropriate manner.

Although the embodiments of the present disclosure have been illustrated and described above, it will be appreciated that the embodiments described above are exemplary and should not be construed as limiting the present disclosure, and that those of ordinary skill in the art can make changes, modifications, replacements, and variations to such embodiments, without departing from the scope of the present disclosure.

## Claims

1. An amplification method, comprising:
S20, providing a surface of a solid carrier, wherein the surface of the solid carrier comprises a first amplification primer, a second amplification primer, and a target polynucleotide that are immobilized thereon, at least a portion of the target polynucleotide complementarily hybridizing with the first amplification primer;
S40, extending the first amplification primer by taking the target polynucleotide as a template and using a first solution or a second solution to obtain an extended strand of the first amplification primer;
S60, replacing the first solution or the second solution with a third solution to remove the target polynucleotide, wherein the third solution contains a denaturing reagent;
S80, replacing the third solution with a fourth solution to allow the extended strand of the first amplification primer to bind to the second amplification primer;
S100, extending the second amplification primer by taking the extended strand of the first amplification primer as a template and replacing the fourth solution with the first solution or the second solution to obtain an extended strand of the second amplification primer;
S120, replacing the first solution or the second solution with the third solution;
S140, replacing the third solution with the fourth solution to allow the extended strand of the first amplification primer and the extended strand of the second amplification primer to bind to the second amplification primer and the first amplification primer, respectively;
S160, extending the first amplification primer and the second amplification primer by replacing the fourth solution with the first solution or the second solution and taking the extended strand of the first amplification primer and the extended strand of the second amplification primer as templates;
S180, repeating S120-S160 at least once,
wherein the first solution, the second solution, and the fourth solution all comprise at least one of betaine, Mg²⁺, NH₄⁺, and formamide, wherein the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume less than or equal to 15%.

2. The amplification method according to claim 1, wherein
the betaine has a concentration greater than 0 mol/L and less than or equal to 3 mol/L in the first solution;
optionally,the betaine has a concentration of 1.5-2.5 mol/L in the second solution;
optionally,the betaine has a concentration of 1.5-2.5 mol/L in the fourth solution;
optionally,the first solution, the second solution, and the fourth solution all comprise DMSO, the DMSO having a percent concentration by volume of 1-5% in each of the first solution, the second solution, and the fourth solution.

3. The amplification method according to claim 1 or 2, wherein
in S40, the first amplification primer is extended by using the first solution, the Mg²⁺ having a concentration of 2-4 mmol/L in the first solution, and the betaine having a concentration of 0.1-2 mol/L in the first solution.

4. The amplification method according to claim 3, wherein
in S100 or S160, the fourth solution is replaced with the second solution, the Mg²⁺ having a concentration of 2-4 mmol/L in the second solution, and the betaine having a concentration of 0.1-2 mol/L in the second solution.

5. The amplification method according to claim 3, wherein
in S100 or S160, the fourth solution is replaced with the second solution, the Mg²⁺ having a concentration of 1-1.5 mmol/L in the second solution, and the betaine having a concentration of 2-3 mol/L in the second solution.
optionally,in S40, the first amplification primer is extended by using the second solution, the Mg²⁺ having a concentration of 2-4 mmol/L in the second solution, and the betaine having a concentration of 0.1-2 mol/L in the second solution.
optionally,in S100 or S160, the fourth solution is replaced with the first solution, the Mg²⁺ having a concentration of 2-4 mmol/L in the first solution, and the betaine having a concentration of 0.1-2 mol/L in the first solution;
optionally,
in S100, the fourth solution is replaced with the first solution, the Mg²⁺ having a concentration of 1-1.5 mmol/L in the first solution, and the betaine having a concentration of 2-3 mol/L in the first solution;
optionally, both the first solution and the second solution comprise dNTPs, the dNTPs having a concentration of 20-1600 µmol/L in the first solution and the second solution.

6. The amplification method according to claim 3 or 4, wherein
the first solution or the second solution further comprises dNTPs, the dNTPs comprising dATP, dTTP, dCTP, and dGTP, and a sum of contents of the dATP and the dTTP being equal to a sum of contents of the dGTP and the dCTP.

7. The amplification method according to claim 5, wherein
the second solution or the first solution further comprises dNTPs, the dNTPs comprising dATP, dTTP, dCTP, and dGTP, and a sum of contents of the dATP and the dTTP being less than a sum of contents of the dGTP and the dCTP;
optionally,the NH₄⁺ is NH₄⁺ provided by at least one of ammonium sulfate, ammonium chloride, and ammonium acetate;
optionally,the formamide has a percent concentration by volume greater than or equal to 3% and less than or equal to 15%;
optionally, the first solution, the second solution, and the fourth solution further comprise Trisbase, the Trisbase having a concentration of 10-500 mmol/L in each of the first solution, the second solution, and the fourth solution;
optionally,the Trisbase has a concentration of 10-200 mmol/L in each of the first solution, the second solution, and the fourth solution;
optionally,
the first solution, the second solution, and the fourth solution further comprise a single-stranded binding protein and/or bovine serum albumin;
the single-stranded binding protein has a concentration of 0.01-1 mg/mL in the first solution, the second solution, and the fourth solution;
the bovine serum albumin has a percent concentration by volume of 0.05-2% in the first solution, the second solution, and the fourth solution;
optionally, the first solution, the second solution, and the fourth solution further comprise a surfactant, the surfactant having a percent concentration by volume of 0.01-2% in each of the first solution, the second solution, and the fourth solution;
optionally,the surfactant is a nonionic surfactant;
optionally,the nonionic surfactant is selected from one or more of Tween 20, DD, DDM, and Triton;
optionally, the first solution, the second solution, and the fourth solution further comprise tetramethylammonium chloride or tetraethylammonium chloride, the tetramethylammonium chloride or tetraethylammonium chloride having a concentration of 10-50 mmol/L in each of the first solution, the second solution, and the fourth solution;
optionally,the denaturing reagent is selected from amides;
optionally,the denaturing reagent is formamide, the formamide having a percent concentration by volume of 60-100% in the third solution;
optionally, the first amplification primer and the second amplification primer are oligonucleotides.

8. The amplification method according to any one of claims 1-7, wherein
S160 is performed under a variable-temperature condition, S120 and S140 are performed under a constant-temperature condition, and a reaction temperature of S160 is not lower than reaction temperatures of S120 and S140;
optionally,S160 being performed under the variable-temperature condition comprises:
in S160, performing a first heating on a reaction system of S160 to raise the temperature of the reaction system of S160 to a first temperature, and maintaining the first temperature for a first preset duration;
after the first preset duration, performing a second heating on the reaction system of S160 to raise the temperature of the reaction system of S160 from the first temperature to a second temperature, and maintaining the second temperature for a second preset duration;
optionally, the first temperature is 60 °C-65 °C, and the first preset duration is 1-10 s; and/or,
the second temperature is 80 °C-85 °C, and the second preset duration is 1-10 s;
optionally, the first heating and the second heating have a heating rate of 0.1-5.0 °C/s;
optionally,
S160 being performed under the variable-temperature condition comprises:
in S160, performing at least one pulsed heating on the reaction system of S160 to raise the temperature of the reaction system of S160 to a third temperature,
wherein a duration of one pulse is a third preset duration;
optionally, the third temperature is 60 °C-85 °C, and the third preset duration is 1-10 s;
optionally, the pulsed heating has a heating rate of 0.1-5.0 °C/s;
optionally,
after completion of one pulse and before performing a next pulse on the reaction system of S160, the temperature of the reaction system of S160 is restored to the temperature prior to the pulsed heating.

9. An amplification method, comprising:
S200, providing a surface of a solid carrier, wherein the surface of the solid carrier comprises a first amplification primer, a second amplification primer, and a target polynucleotide that are immobilized thereon, at least a portion of the target polynucleotide complementarily hybridizing with the first amplification primer;
S400, extending the first amplification primer by taking the target polynucleotide as a template and using the first solution or the second solution to obtain an extended strand of the first amplification primer;
S600, replacing the first solution or the second solution with a third solution to remove the target polynucleotide, wherein the third solution contains a denaturing reagent;
S800, replacing the third solution with a fourth solution to allow the extended strand of the first amplification primer to bind to the second amplification primer;
S1000, extending the second amplification primer by taking the extended strand of the first amplification primer as a template and replacing the fourth solution with the first solution or the second solution to obtain an extended strand of the second amplification primer;
S1200, replacing the first solution or the second solution with the third solution;
S1400, replacing the third solution with the fourth solution to allow the extended strand of the first amplification primer and the extended strand of the second amplification primer to bind to the second amplification primer and the first amplification primer, respectively;
S1600, extending the first amplification primer and the second amplification primer by replacing the fourth solution with the first solution or the second solution and taking the extended strand of the first amplification primer and the extended strand of the second amplification primer as templates;
S1800, repeating S1200-S1600 at least once,
wherein S1600 is performed under a variable-temperature condition, S1200 and S1400 are performed under a constant-temperature condition, and a reaction temperature of S1600 is not lower than reaction temperatures of S1200 and S1400.

10. The amplification method according to claim 9, wherein
S1600 being performed under the variable-temperature condition comprises:
in S1600, performing a first heating on a reaction system of S1600 to raise the temperature of the reaction system of S1600 to a first temperature, and maintaining the first temperature for a first preset duration;
after the first preset duration, performing a second heating on the reaction system of S1600 to raise the temperature of the reaction system of S1600 from the first temperature to a second temperature, and maintaining the second temperature for a second preset duration;
optionally, the first temperature is 60 °C-65 °C, and the first preset duration is 1-10 s;
the second temperature is 80 °C-85 °C, and the second preset duration is 1-10 s;
optionally, the first heating and the second heating have a heating rate of 0.1-5.0 °C/s.

11. The amplification method according to claim 9 or 10, wherein
S1600 being performed under the variable-temperature condition comprises:
in S1600, performing at least one pulsed heating on the reaction system of S1600 to raise the temperature of the reaction system of S1600 to a third temperature,
wherein a duration of one pulse is a third preset duration;
optionally, the third temperature is 60 °C-85 °C, and the third preset duration is 1-10 s;
optionally, the pulsed heating has a heating rate of 0.1-5.0 °C/s.

12. The amplification method according to any one of claims 9-11, wherein
after completion of one pulse and before performing a next pulse on the reaction system of S1600, the temperature of the reaction system of S1600 is restored to the temperature prior to the pulsed heating;
optionally, the first solution, the second solution, and the fourth solution all comprise at least one of betaine, Mg²⁺, NH₄⁺, and formamide, wherein the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume less than or equal to 15%;
optionally,the betaine has a concentration greater than 0 mol/L and less than or equal to 3 mol/L in the first solution;
optionally,the betaine has a concentration of 1.5-2.5 mol/L in the second solution;
optionally, the betaine has a concentration of 1.5-2.5 mol/L in the fourth solution;
optionally,
the first solution, the second solution, and the fourth solution all comprise DMSO, the DMSO having a percent concentration by volume of 1-5% in each of the first solution, the second solution, and the fourth solution;
optionally,in S400, the first amplification primer is extended by using the first solution, the Mg²⁺ having a concentration of 2-4 mmol/L in the first solution, and the betaine having a concentration of 0.1-2 mol/L in the first solution;
optionally,in S1000 or S1600, the fourth solution is replaced with the second solution, the Mg²⁺ having a concentration of 2-4 mmol/L in the second solution, and the betaine having a concentration of 0.1-2 mol/L in the second solution;
optionally,in S1000 or S1600, the fourth solution is replaced with the second solution, the Mg²⁺ having a concentration of 1-1.5 mmol/L in the second solution, and the betaine having a concentration of 2-3 mol/L in the second solution;
optionally,in S400, the first amplification primer is extended by using the second solution, the Mg²⁺ having a concentration of 2-4 mmol/L in the second solution, and the betaine having a concentration of 0.1-2 mol/L in the second solution;
optionally,in S1000 or S1600, the second amplification primer is extended by replacing the fourth solution with the first solution, the Mg²⁺ having a concentration of 2-4 mmol/L in the first solution, and the betaine having a concentration of 0.1-2 mol/L in the first solution;
optionally,in S1000 or S1600, the second amplification primer is extended by replacing the fourth solution with the first solution, the Mg²⁺ having a concentration of 1-1.5 mmol/L in the first solution, and the betaine having a concentration of 2-3 mol/L in the first solution. 54. The amplification method according to any one of claims 35-53, wherein
both the first solution and the second solution comprise dNTPs, the dNTPs having a concentration of 20-1600 µmol/L in the first solution and the second solution;
optionally,the first solution or the second solution further comprises dNTPs, the dNTPs comprising dATP, dTTP, dCTP, and dGTP, and a sum of contents of the dATP and the dTTP being equal to a sum of contents of the dGTP and the dCTP;
optionally,the second solution or the first solution further comprises dNTPs, the dNTPs comprising dATP, dTTP, dCTP, and dGTP, and a sum of contents of the dATP and the dTTP being less than a sum of contents of the dGTP and the dCTP;
optionally,the NH₄⁺ is NH₄⁺ provided by at least one of ammonium sulfate, ammonium chloride, and ammonium acetate;
optionally, the first solution, the second solution, and the fourth solution further comprise Trisbase, the Trisbase having a concentration of 10-500 mmol/L in each of the first solution, the second solution, and the fourth solution;
optionally,the Trisbase has a concentration of 10-200 mmol/L in each of the first solution, the second solution, and the fourth solution;
optionally, the first solution, the second solution, and the fourth solution further comprise a single-stranded binding protein and/or bovine serum albumin;
the single-stranded binding protein has a concentration of 0.01-1 mg/mL in the first solution, the second solution, and the fourth solution;
the bovine serum albumin has a percent concentration by volume of 0.05-2% in the first solution, the second solution, and the fourth solution;
optionally, the first solution, the second solution, and the fourth solution further comprise a surfactant, the surfactant having a percent concentration by volume of 0.01-2% in each of the first solution, the second solution, and the fourth solution;
optionally, the surfactant is a nonionic surfactant;
optionally,the nonionic surfactant is selected from one or more of Tween 20, DD, DDM, and Triton;
optionally, the first solution, the second solution, and the fourth solution further comprise tetramethylammonium chloride or tetraethylammonium chloride, the tetramethylammonium chloride or tetraethylammonium chloride having a concentration of 10-50 mmol/L in each of the first solution, the second solution, and the fourth solution;
optionally,the denaturing reagent is selected from amides;
optionally,the denaturing reagent is formamide, the formamide having a percent concentration by volume of 60-100% in the third solution;
optionally, the first amplification primer and the second amplification primer are oligonucleotides.

13. An amplification method, comprising:
S2000, providing a surface of a solid carrier, wherein the surface of the solid carrier comprises a first amplification primer, a second amplification primer, and a target polynucleotide that are immobilized thereon, at least a portion of the target polynucleotide complementarily hybridizing with the first amplification primer;
S4000, extending the first amplification primer by taking the target polynucleotide as a template and using a first solution or a second solution to obtain an extended strand of the first amplification primer;
S6000, replacing the first solution or the second solution with a third solution to remove the target polynucleotide, wherein the third solution contains a denaturing reagent;
S8000, replacing the third solution with a fourth solution to allow the extended strand of the first amplification primer to bind to the second amplification primer;
S10000, extending the second amplification primer by taking the extended strand of the first amplification primer as a template and replacing the fourth solution with the first solution or the second solution to obtain an extended strand of the second amplification primer;
S12000, replacing the first solution or the second solution with the third solution;
S14000, replacing the third solution with the fourth solution to allow the extended strand of the first amplification primer and the extended strand of the second amplification primer to bind to the second amplification primer and the first amplification primer, respectively;
S16000, extending the first amplification primer and the second amplification primer by replacing the fourth solution with the first solution or the second solution and taking the extended strand of the first amplification primer and the extended strand of the second amplification primer as templates;
S18000, repeating S12000-S16000 at least once,
wherein in S10000 and S16000, the first solution or the second solution comprises dATP, dTTP, dCTP, and dGTP, and a sum of contents of the dATP and the dTTP is less than a sum of contents of the dGTP and the dCTP.

14. The amplification method according to claim 13, wherein
the first solution, the second solution, and the fourth solution all comprise at least one of betaine, Mg²⁺, NH₄⁺, and formamide, wherein the betaine has a concentration greater than 0 mol/L and less than or equal to 4 mol/L, the Mg²⁺ has a concentration of 0.5-4 mmol/L, the NH₄⁺ has a concentration greater than 0 mmol/L and less than or equal to 100 mmol/L, and the formamide has a percent concentration by volume less than or equal to 15%.

15. The amplification method according to claim 14, wherein
in S4000, the first amplification primer is extended by using the first solution, and in S10000 and S16000, the fourth solution is replaced with the first solution or the second solution, wherein in the first solution, the Mg²⁺ has a concentration of 2-4 mmol/L, and the betaine has a concentration of 0.1-2 mol/L, and in the second solution, the Mg²⁺ has a concentration of 2-4 mmol/L, and the betaine has a concentration of 0.1-2 mol/L; or
in S4000, the first amplification primer is extended by using the first solution, and in S10000 and S16000, the fourth solution is replaced with the first solution or the second solution, wherein in the first solution, the Mg²⁺ has a concentration of 2-4 mmol/L, and the betaine has a concentration of 0.1-2 mol/L, and in the second solution, the Mg²⁺ has a concentration of 1-1.55 mmol/L, and the betaine has a concentration of 2-3 mol/L;
optionally,in S4000, the first amplification primer is extended by using the second solution; and in S10000 and S16000, the fourth solution is replaced with the first solution or the second solution, wherein in the first solution, the Mg²⁺ has a concentration of 2-4 mmol/L, and the betaine has a concentration of 0.1-2 mol/L, and in the second solution, the Mg²⁺ has a concentration of 2-4 mmol/L, and the betaine has a concentration of 0.1-2 mol/L;
optionally,in S4000, the first amplification primer is extended by using the second solution; and in S10000 and S16000, the second amplification primer is extended by replacing the fourth solution with the first solution or the second solution, wherein in the first solution, the Mg²⁺ has a concentration of 1-1.5 mmol/L, and the betaine has a concentration of 2-3 mol/L, and in the second solution, the Mg²⁺ has a concentration of 2-4 mmol/L, and the betaine has a concentration of 0.1-2 mol/L;
optionally,in S4000, the first solution or the second solution comprises dATP, dTTP, dCTP, and dGTP, and a sum of contents of the dATP and the dTTP is equal to a sum of contents of the dGTP and the dCTP;
optionally,the betaine has a concentration of 1.5-2.5 mol/L in the fourth solution;
optionally,the NH₄⁺ is NH₄⁺ provided by at least one of ammonium sulfate, ammonium chloride, and ammonium acetate;
optionally, the first solution, the second solution, and the fourth solution further comprise Trisbase, the Trisbase having a concentration of 10-500 mmol/L in each of the first solution, the second solution, and the fourth solution;
optionally,the Trisbase has a concentration of 10-200 mmol/L in each of the first solution, the second solution, and the fourth solution;
optionally, the first solution, the second solution, and the fourth solution further comprise a single-stranded binding protein and/or bovine serum albumin;
the single-stranded binding protein has a concentration of 0.01-1 mg/mL in the first solution, the second solution, and the fourth solution;
the bovine serum albumin has a percent concentration by volume of 0.05-2% in the first solution, the second solution, and the fourth solution;
optionally,
the first solution, the second solution, and the fourth solution further comprise a surfactant, the surfactant having a percent concentration by volume of 0.01-2% in each of the first solution, the second solution, and the fourth solution;
optionally, the surfactant is a nonionic surfactant;
optionally,
the nonionic surfactant is selected from one or more of Tween 20, DD, DDM, and Triton;
optionally, the first solution, the second solution, and the fourth solution further comprise tetramethylammonium chloride or tetraethylammonium chloride, the tetramethylammonium chloride or tetraethylammonium chloride having a concentration of 10-50 mmol/L in each of the first solution, the second solution, and the fourth solution;
optionally,the denaturing reagent is selected from amides;
optionally,the denaturing reagent is formamide, the formamide having a percent concentration by volume of 60-100% in the third solution;
optionally, the first amplification primer and the second amplification primer are oligonucleotides;
optionally,S16000 is performed under a variable-temperature condition, S12000 and S14000 are performed under a constant-temperature condition, and a reaction temperature of S16000 is not lower than reaction temperatures of S12000 and S14000;
optionally,S16000 being performed under the variable-temperature condition comprises:
in S16000, performing a first heating on a reaction system of S16000 to raise the temperature of the reaction system of S16000 to a first temperature, and maintaining the first temperature for a first preset duration;
after the first preset duration, performing a second heating on the reaction system of S16000 to raise the temperature of the reaction system of S16000 from the first temperature to a second temperature, and maintaining the second temperature for a second preset duration;
optionally, the first temperature is 60 °C-65 °C, and the first preset duration is 1-10 s;
the second temperature is 80 °C-85 °C, and the second preset duration is 1-10 s;
optionally, the first heating and the second heating have a heating rate of 0.1-5.0 °C/s;
optionally,S16000 being performed under the variable-temperature condition comprises:
in S16000, performing at least one pulsed heating on the reaction system of S16000 to raise the temperature of the reaction system of S16000 to a third temperature,
wherein a duration of one pulse is a third preset duration;
optionally, the third temperature is 60 °C-85 °C, and the third preset duration is 1-10 s;
optionally,the pulsed heating has a heating rate of 0.1-5.0 °C/s;
optionally,after completion of one pulse and before performing a next pulse on the reaction system of S16000, the temperature of the reaction system of S16000 is restored to the temperature prior to the pulsed heating.
